# FASCICULE DE BREVET EUROPEEN

(11) **EP 1 664 334 B1**
(45) Date de publication et mention de la délivrance du brevet: **18.04.2012**
(21) Numéro de dépôt: 04787169.4
(22) Date de dépôt: 17.09.2004
(51) Int. Cl.: C12Q 1/02, C12Q 1/48, G01K 3/04, G01N 33/02

(54) **PROCEDE ET DISPOSITIF POUR DETERMINER SI UN PRODUIT EST EN ETAT D'ETRE UTILISE OU CONSOMME**
VERFAHREN UND VORRICHTUNG ZUR BESTIMMUNG, OB EIN PRODUKT ZUR VERWENDUNG ODER ZUM VERBRAUCH GEEIGNET IST
METHOD AND DEVICE FOR DETERMINING IF A PRODUCT IS IN CONDITION FOR USE OR CONSUMPTION

(30) Priorité: 17.09.2003 FR 0350556; 03.02.2004 FR 0450200
(43) Date de publication de la demande: 07.06.2006
(73) Titulaire: Cryolog S.A., 92100 Boulogne (FR)
(72) Inventeur: LOUVET, Olivier, F-67000 Strasbourg (FR); THUAULT, Dominique, F-29170 Pleuven (FR); VAILLANT, Renaud, F-94250 Gentilly (FR)
(74) Mandataire: Larcher, Dominique
(86) Numéro de dépôt international: PCT/EP2004/052237
(87) Numéro de publication internationale: WO 2005/026383

(56) Documents cités:
- EP-A1- 0 497 459
- WO-A-92/14998
- WO-A-03/025529
- US-A- 2 950 202
- US-A- 5 053 339
- SAMELIS J.; GEORGIADOU K.G.: "The microbial association of Greek taverna sausage stored at 4 and 10 degrees C in air, vacuum or 100% carbon dioxide, and its spoilage potential", JOURNAL OF APPLIED MICROBIOLOGY, vol. 88, January 2000 (2000-01), pages 58-68, ENGLAND
- BARAKAT R.K.; GRIFFITHS M.W.; HARRIS L.J.: "Isolation and characterization of Carnobacterium, Lactococcus, and Enterococcus spp. from cooked, modified atmosphere packaged, refrigerated, poultry meat", INTERNATIONAL JOURNAL OF FOOD MICROBIOLOGY, vol. 62, 5 December 2000 (2000-12-05), pages 83-94,
- HAMASAKI YOSHIKATSU, ET AL: 'Behavior of psychrotrophic lactic acid bacteria isolated from spoiling cooked meat products' APPLIED AND ENVIROMENTAL MICROBIOLOGY vol. 69, no. 6, 01 Juin 2003, USA, pages 3668 - 3671

## Description

L'invention est relative à un procédé et à un indicateur pour déterminer si un produit est ou non en condition d'être utilisé ou consommé, ce produit étant destiné à être conservé dans des conditions de température et pendant une durée limitant sa dégradation.

Dans la demande de brevet internationale publiée sous le numéro WO03/025529, on a décrit un procédé pour contrôler de manière systématique et automatique un produit dont la conservation dans un circuit de distribution dépend de la température, notamment un produit soumis à une chaîne du froid.

Dans le procédé décrit dans cette demande antérieure, il est prévu un marqueur qui change d'état lorsque la température du produit franchit un seuil de température déterminée, ou lorsque les conditions de conservation s'écartent d'un niveau de référence déterminé, ces conditions étant soit le franchissement d'un seuil de température déterminée pendant une durée supérieure à une durée déterminée, soit le franchissement d'une durée de conservation déterminée.

Autrement dit, la technique décrite dans ce document antérieur prévoit des indications qui sont fonction de franchissement de seuils ou limites de temps ou de température déterminés.

On connaît également la technique décrite dans la demande EP-A1-0497459 qui concerne un dispositif permettant de renseigner le consommateur sur d'éventuels dépassements de la période de consommation ou de la température de stockage d'un produit, ces variations étant susceptibles d'altérer la qualité du produit alimentaire. Ce dispositif est basé sur l'utilisation d'un indicateur pouvant comprendre des microorganismes acidifiants. Le changement de pH provoque alors un changement de couleur de l'indicateur, avertissant le consommateur d'un changement de température et d'une altération de la qualité du produit.

Les produits thermosensibles, et notamment les produits alimentaires, sont des produits qui évoluent de manière continue en fonction du couple temps température. L'existence d'une température de conservation n'est qu'une recommandation permettant de garantir une durée d'utilisation du produit.

C'est le cas notamment des produits alimentaires frais.

En fait, les normes, notamment celles relatives aux produits alimentaires, imposent une date limite de consommation ou d'utilisation qui dépend des conditions de température et de temps de conservation. Par exemple, pour un produit alimentaire devant être conservé entre 0°C et +4°C, la date limite de consommation déterminé par les autorités intègre l'exposition du produit, pendant une période plus ou moins longue, à une température supérieure (+8°C) correspondant à une rupture de chaîne du froid raisonnable (norme AFNOR NF V 01-003).

L'invention part de la constatation que les dates limites d'utilisation ne sont en général pas optimales car elles sont déterminées sur des conditions de conservation présupposées. Par exemple, pour un produit périssable devant être conservé entre 0°C et +4°C, sa durée d'utilisation peut être étendue par rapport à ce qu'indique la norme si le produit n'est pas exposé à des températures supérieures à +4°C, dans la mesure où ladite norme prévoit une exposition à une température de rupture de chaîne du froid de +8°C. Ceci signifie que certains produits ne sont pas consommés alors qu'ils pourraient l'être, ce qui constitue un inconvénient important tant en termes pratiques qu'économiques.

Ainsi, dans le procédé selon l'invention, on associe au produit périssable un indicateur présentant des caractéristiques d'évolution en fonction du temps et de la température, analogues aux caractéristiques correspondantes du produit. Cet indicateur étant tel qu'il fournit une information sur la possibilité d'utilisation ou de consommation d'un produit périssable en fonction de l'évolution des conditions de conservation du produit ;
de sorte que l'indicateur fournit une information permettant que le produit soit utilisable ou consommable pendant une période plus importante si le produit a été conservé dans des conditions meilleures que des conditions standards,
et de sorte que, l'indicateur fournit une information permettant que le produit soit utilisable ou consommable pendant une période plus courte si le produit a été conservé dans des conditions moins bonnes que les conditions standards.

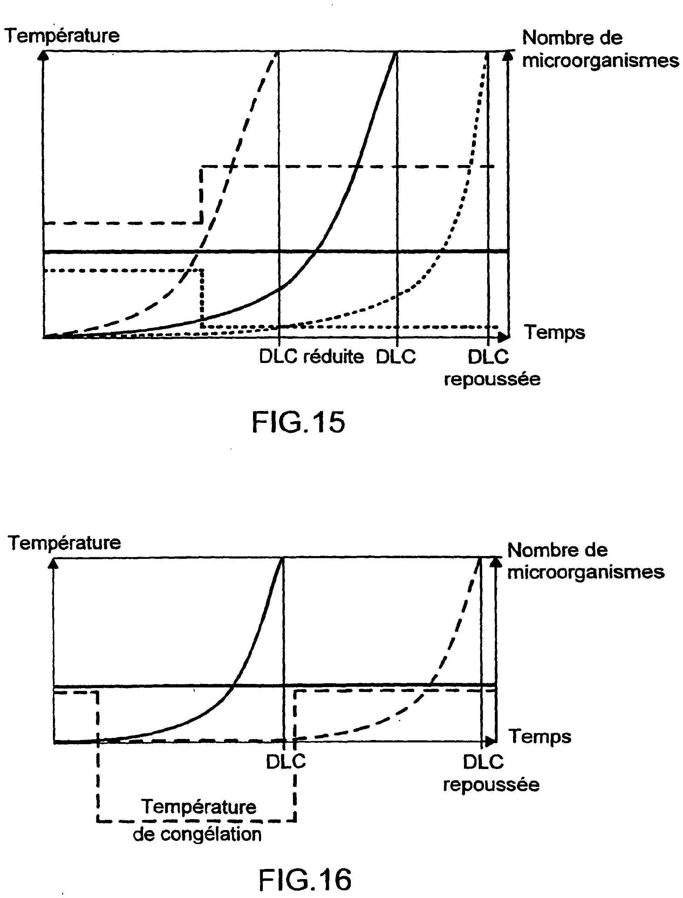

Ainsi, avec ce procédé, la date limite d'utilisation ou de consommation varie en fonction des conditions de température et de la durée de conservation réelles du produit ; la date limite de consommation (DLC) ou la date limite d'utilisation optimale (DLUO) inscrite par l'industriel au moment du conditionnement du produit périssable, devient dynamique et évolue alors en fonction des conditions réelles du produit. Par exemple, pour un produit alimentaire, la durée de conservation sera réduite si les conditions de température se dégradent et, par contre, la date limite de consommation sera repoussée si les conditions de température sont meilleures, ainsi que l'illustre la Figure 15.

Dans ces conditions, la date limite de consommation peut être considérablement allongée par rapport à celle prévue par les règlements. A titre d'exemple, un produit frais ayant une date limite de consommation déterminée, peut-être congelé par le consommateur ; une fois congelé, le produit pourra être conservé pendant plusieurs semaines voire plusieurs mois, et pourra donc être consommé bien au-delà de la date limite de consommation indiquée sur la produit périssable, tel qu'illustré dans la Figure 16. Le procédé, tel que décrit, prend en compte la dégradation réelle du produit avant congélation, n'évolue plus pendant la phase de congélation et indique après décongélation que le produit est consommable, jusqu'à l'indication de changement d'état, information dont le consommateur ne dispose pas aujourd'hui.

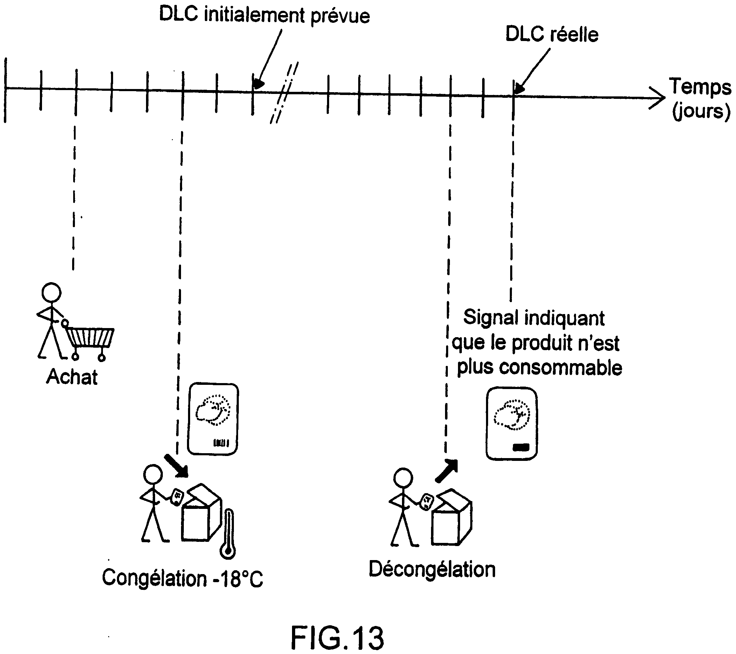

Supposons qu'un consommateur achète un produit périssable dont la date limite de consommation est de 7 jours après la date de conditionnement, dans les conditions normales de conservation. Supposons qu'il décide de congeler le produit 2 jours avant l'expiration de la DLC. La congélation à -18°C stoppant la dégradation microbiologique du produit, le consommateur, peut décider un mois plus tard de décongeler son produit et de le consommer dans les jours suivants. Le consommateur ne disposera alors d'aucune information lui permettant de savoir jusqu'à quand il peut consommer le produit périssable. Le procédé tel que décrit permet de répondre à cette problématique puisqu'il prend en compte la dégradation réelle du produit avant, pendant et après la congélation. Ainsi le consommateur disposera d'un indicateur qui, en l'absence de changement d'état, lui confirmera qu'il peut consommer le produit ; et qui lui signalera, par un changement d'état, que le produit ne doit plus être consommée, si le consommateur conserve le produit trop longtemps dans son réfrigérateur après décongélation. Ceci est illustré dans la Figure 13.

En outre, on sait qu'il est habituellement fortement déconseillé de recongeler un produit qui vient d'être décongelé, notamment en raison du risque de développement des bactéries qui étaient initialement présentes dans le produit. Avec l'invention, il est également possible de maîtriser ce risque puisque l'indicateur présente des caractéristiques d'évolution, en fonction du temps et de la température, analogues aux caractéristiques correspondantes du produit. Ainsi, l'indicateur suivra la même évolution que le produit périssable et, pourra être recongelé, le changement d'état de l'indicateur intégrant alors l'ensemble des périodes durant lesquelles le produit est exposé à des températures entraînant sa dégradation.

Toutefois, l'invention n'est pas limitée aux produits alimentaires. D'autres produits, tels que les médicaments, les vaccins, le sang à transfuser, doivent être conservés dans des grammes prédéterminées de températures et le fait de sortir de la gamme (par le haut ou par le bas) pourra amener à avancer la date limite d'utilisation. De même, si le produit est conservé dans des conditions optimales, la date limite d'utilisation pourra être allongée.

De façon générale, si le produit, bien que devant être conservé dans une gamme déterminée de températures, présente une température optimale de conservation, alors la date limite de consommation ou d'utilisation sera d'autant repoussée qu'on se rapprochera de cette température optimale.

Il est à noter que par rapport aux techniques antérieures connues, notamment celles relatives à la conservation des produits alimentaires, l'invention n'est pas limitée au fait que l'indicateur soit utile lorsque la température de conservation du produit dépasse une limite, mais s'applique dans les conditions normales de conservation du produit. L'invention est donc plus qu'un simple témoin de rupture de la chaîne du froid ou qu'un simple indicateur de franchissement de seuil. C'est plutôt une véritable date limite de consommation dynamique, ou date limite d'utilisation dynamique, d'un produit périssable.

De préférence, on fournit un indicateur contenant une substance microbienne, notamment quand le produit périssable concerné est un aliment ou un produit biologique.

Dans une réalisation, on fournit un indicateur à substance microbienne produisant un acide et on mesure l'activité de cette substance microbienne en suivant l'évolution du pH du milieu, en général une acidification ; de sorte que, tant que le pH n'est pas descendu en dessous d'une valeur prédéterminée, l'indicateur signale que le produit est consommable ; et de sorte que, lorsque le pH descend en dessous d'une valeur prédéterminée, l'indicateur signale que le produit n'est plus consommable.

Dans ce cas, pour suivre l'évolution du pH dans l'indicateur, on ajoute, par exemple, au milieu contenant la substance microbienne de la caséine qui forme un précipité lorsque le pH descend au-dessous de la valeur déterminée ce qui modifie l'aspect du milieu.

Ainsi, l'invention concerne un procédé pour déterminer si un produit est ou non en état d'être utilisé ou consommé, ce produit étant destiné à être conservé dans des conditions de température et pendant une durée limitant sa dégradation, procédé dans lequel on associe au produit un indicateur tel qu'il fournit un signal indiquant la possibilité d'utilisation ou de consommation du produit, puis, après l'expiration d'une durée de conservation, un signal indiquant un changement d'état de possibilité d'utilisation ou de consommation du produit, cette durée évoluant en fonction des conditions de conservation du produit,
de sorte que l'indicateur fournit une information émettant que le produit soit utilisable ou consommable pendant une durée plus importante si le produit a été conservé dans des conditions meilleures que des conditions standards ou préconisées, ou pendant une durée moins importante si le produit a été conservé dans des conditions moins bonnes que des conditions standards ou préconisées, l'indicateur contenant au moins une substance microbienne ayant une activité acidifiante faisant varier le pH du milieu dans lequel elle se trouve
ladite substance microbienne contenant au moins une souche de bactéries choisie parmi :
- la souche CRYO-CB001 de l'espèce *Carnobacterium piscicola* enregistrée auprès de la CNCM (Collection Nationale de Culture de Microorganismes, Paris, France) sous le N° CNCM I-3297, le 16 septembre 2004 ; et
- la souche CRYO-CB002 de l'espèce *Carnobacterium piscicola* enregistrée auprès de la CNCM (Collection Nationale de Culture de Microorganismes, Paris, France) sous le N° CNCM I-3298, le 16 septembre 2004 ;
et dans lequel on produit, à l'aide d'un révélateur, le signal indiquant le changement d'état quand le pH du milieu dans lequel se trouve la substance microbienne descend au dessous d'une valeur prédéterminée.

Dans une réalisation, le révélateur forme un précipité lorsque le pH descend au-dessous de la valeur prédéterminée.

Ce révélateur comporte par exemple de la caséine.

Dans une réalisation le révélateur comporte au moins un indicateur coloré.

L'indicateur coloré est par exemple choisi dans le groupe suivant : Alizarine, Alizarine Sodium Sulfonate, Alzarine Red, Benzopurpurin, Benzol Auramine G, Benzoylethylauramine, Bleu De Résorcine, Bleu De Bromochlorophénol, Bromocresol Green, Bromophenol Blue, Carmine Acid, 2,4-Dinitrophenol, 4-(4-Dimethylamino-1-Naphtylazo)-3-Methoxybenzenesulfonic Acid, α-dinitrophénol, β-dinitrophénol, γ-dinitrophénol, Disodium4,4-Bis(O-Tolytriazeno)-2,2'- Stilbenedisulfonate, Disodium4,4-Bis (P-Dimethylaminophenylazo) -2,2'-Stilbenedisulfonate, 4- (P-Ethoxyphenylazo)-M-Phenylene-Diamine Monohydrochloride, Ethyl Red, Ethyl Orange, Hexamthoxy Red, Hydroquinolsulphonaphtalein, Lacmoid, Iodeosin, Lasmoid, N,Ndimethyl-p-(M-Tolylazo)Aniline, 4'-Methoxy-2.4-Diaminoazabenzene, Methyl Red, Methyl Red-Alphazurin, α-Naphtylamine, α-Naphtylminoazo-Benzène, Naphtyl Red, Oxime Blue, 4'-Oxy-3'Methyl-2.4-Diaminoazobenzene, 4'-Oxy-2.4diaminoazobenzène, 4-Phenylazo-1-Naphtylamine, Parafuchsine-Hexa-Acetic Acid, Paranitrophénol, P-Ethoxychrysoidine, P-Sulfo-O-Methoxybenzeneazodimethyl- αNaphtylamine, Rouge Congo, Red Violet, Resazurin, Rouge Naphtyl, Tetrabromocresol, Tétraiodophenosulfophtlalein, acide aurine, dithiozone, bleu de chrome, bromocrésol pourpre, carmin indigo, cacotheline, calcéine, eosine extra, eriochromocyanine, jaune thiazol, noir d'eriochrome T, α-nitro β-naphtol, orange III, rouge methyl, rhodamine B**,** rhodizonate de potassium, thymolsulffonephtalein, rhodium chlorure, trihydroxy2,6,7phenyl9 isoxanthen, thorin, xylene orange tetranatrium salz, anthocyanes, phénolphtaléine, le benzopurpurin 4B, benzopurpurin B, alpha naphtyl red hydrochloride, litmus, Rouge de méthyle, Indicateur Mixte, Lacmoïd.

Dans une réalisation, pour adapter l'indicateur au produit, on choisit au moins un des paramètres compris dans le groupe suivant : la nature de la substance microbienne, la quantité de cette substance microbienne, la nature des nutriments, la quantité des nutriments, la nature des éléments nécessaires à la production d'acide par la substance microbienne et la quantité de ces éléments, la nature d'un texturant du milieu dans lequel se trouve la substance microbienne et la quantité de ce texturant, le pH de départ du milieu, les paramètres influant sur l'a_{w} du milieu, la nature du révélateur utilisé pour mesurer la baisse du pH et la quantité de ce révélateur, les paramètres déterminant le pH auquel ce révélateur change d'état.

Les paramètres sont par exemple choisis pour que le signal indiquant le changement d'état ait lieu au bout d'une durée prédéterminée lorsque le produit est conservé dans des conditions standards ou préconisées.

Les paramètres peuvent être choisis pour que le signal indiquant le chargement d'état apparaisse lorsque le produit n'est plus consommable.

Dans une réalisation, dans des conditions standards ou préconisées, on choisit les paramètres de l'indicateur pour que le signal indiquant le changement d'état apparaisse après une durée déterminée, cette durée étant augmentée en modifiant un de ces paramètres en l'éloignant des valeurs optimales pour l'acidification par la substance microbienne.

Dans une réalisation, les nutriments incluent une source de carbone et une source d'azote, et, de préférence, des sels minéraux et/ou des vitamines et/ou des oligoéléments.

La source de carbone est par exemple choisie parmi les sucres du groupe suivant : Glycérol, Erythritol,D-Arabinose, L-Arabinose,Ribose, D-Xylose, L-Xylose, Adonitol, β Methyl-xyloside, Galactose, D-Glucose, D-Fructose, D-Mannose, L-Sorbose, Rhamnose, Dulcitol, Inositol, Mannitol, Sorbitol, α Methyl-D-mannoside, α Methyl-D-glucoside, N Acetyl glucosamine, Amygdaline, Arbutine, Esculine, Salicine, Cellobiose, Maltose, Lactose, Melibiose, Saccharose, Trehalose, Inuline, Melezitose,
D--Raffinose, Amidon, Glycogène, Xylitol, βGentiobiose, D-Turanose, D-Lyxose, D-Tagatose, D-Fucose, L-Fucose, D-Arabitol, L-Arabitol, Gluconate, 2 ceto-gluconate, 5 ceto-gluconate.

Le texturant est par exemple choisi parmi ceux du groupe suivant : agar, agarose, gélatine, xanthane, scléroglucane, Gum Guar.

Dans une réalisation, l'indicateur est inactivé quand il n'est pas associé au produit et activé lorsqu'il est associé au produit.

L'inactivation est par exemple réalisable à l'aide de moyens choisis dans le groupe suivant : congélation, microencapsulation de la substance microbienne et/ou des nutriments, compartimentation.

Dans une réalisation, l'activation de l'indicateur est réalisée par une action physique choisie dans le groupe suivant : variation de pression, variation de température, Variation de longueur d'onde d'exposition à un rayonnement.

Dans une réalisation, la substance microbienne peut être congelée ou décongelée.

L'invention concerne aussi l'application du procédé défini ci-dessus à des produits de type alimentaire ou biologique ou pharmaceutique.

L'invention concerne également l'application du procédé défini ci-dessus à des produits alimentaires dans lequel l'indicateur est paramétré pour que l'apparition du signal indiquant un changement d'état soit d'autant plus retardée que la température de conservation du produit est proche de la température optimale de conservation.

L'invention concerne aussi l'application du procédé défini ci-dessus à un produit alimentaire destiné à être conservé à des températures comprises entre 0°C et +4°C environ, et dans lequel l'indicateur comprend une substance microbienne ayant une activité acidifiante à des températures inférieures ou égales à +4°C environ.

L'invention concerne également un dispositif pour déterminer si un produit est ou non en état d'être utilisé ou consommé, ce produit étant destiné à être conservé dans des conditions de température et pendant une durée limitant sa dégradation, ce dispositif comprenant au moins un indicateur destiné à être associé au produit, cet indicateur étant tel qu'il fournit un signal indiquant la possibilité d'utilisation ou de consommation du produit, puis, après l'expiration d'une durée de conservation, un signal indiquant un changement d'état de possibilité d'utilisation ou de consommation du produit, cette durée évoluant en fonction des conditions de conservation du produit,
de sorte que l'indicateur fournit une information permettant que le produit soit utilisable ou consommable pendant une durée plus importante si le produit a été conservé dans des conditions meilleures que des conditions standards ou préconisées, ou pendant une durée moins importante si le produit a été conservé dans des conditions moins bonnes que des conditions standards ou préconisées, ou pendant une durée moins importante si le produit a été conservé dans des conditions moins bonnes que des conditions standards ou préconisées, l'indicateur contenant au moins une substance microbienne ayant une activité acidifiante faisant varier le pH du milieu dans lequel elle se trouve,
ladite substance microbienne contenant au moins une souche de bactéries choisie parmi :
- la souche CRYO-CB001 de l'espèce *Carnobacterium piscicola* enregistrée auprès de la CNCM (Collection Nationale de Culture de Microorganismes, Paris, France) sous le N° CNCM I-3297, le 16 septembre 2004 ; et
- la souche CRYO-CB002 de l'espèce *Carnobacterium piscicola* enregistrée auprès de la CNCM (Collection Nationale de Culture de Microorganismes, Paris, France) sous le N° CNCM I-3298, le 16 septembre 2004 ;
ledit indicateur contenant en outre, un révélateur et des moyens pour fournir un signal indiquant le changement d'état quand le pH du milieu dans lequel se trouve la substance microbienne descend au-dessous d'une valeur prédéterminée.

Le révélateur est par exemple tel qu'il forme un précipité lorsque le pH descend au-dessous de la valeur prédéterminée.

Dans une réalisation, le révélateur comporte de la caséine.

Dans une réalisation, le révélateur comporte au moins un indicateur coloré.

Dans une réalisation, l'indicateur coloré est compris dans le groupe suivant : Alizarine, Alizarine Sodium Sulfonate, Alzarine Red ,Benzopurpurin, Benzoyl Auramine *G*, Benzoylethylauramine, Bleu De Résorcine, Bleu De Bromochlorophénol, Bromocresol Green, Bromophenol Blue, Carmine Acid, 2,4-Dinitrophenol, 4-(4-Dimethylamino-1-Naphtylazo)-3-Methoxybenzenesulfonic Acid, α-dinitrophénol, β-dinitrophénol, γ-dinitrophénol, Disodium4,4- Bis(O-Tolytriazeno)-2,2'-Stilbenedisulfonate, Disodium4,4- Bis(P-Dimethylaminophenylazo) - 2,2'-Stilbenedisulfonate, 4-(P-Ethoxyphenylazo)-M-Phenylene-Diamine Monohydrochloride, Ethyl Red, Ethyl Orange, Hexamethoxy Red, Hydroquinolsulphonaphtalein, Lacmoid, Iodeosin, Lasmoid, N,Ndimethyl-P-(M-Tolylazo)Aniline, 4'-Methoxy-2.4-Diaminoazobenzene, Methyl Red, Methyl Red-Alphazurin, α-Naphtylamine, α-Naphtylaminoazo-Benzène, Naphtyl Red, Oxime Blue, 4'-Oxy-3'-Methyl-2.4-Diaminoazobenzene, 4'-Oxy-2.4diaminoazobenzène, 4-Phenylazo-1-Naphtylamine, Parafuchsine-Hexa-Acetic Acid, Paranitrophénol, P-Ethoxychrysoidine, P-Sulfo-O-Methoxybenzeneazodimethyl- αNaphtylamine, Rouge Congo, Red Violet, Resazurin, Rouge Naphtyl, Tetrabromocresol, Tétraiodophenolsulfophthalein, acide aurine, dithiozone, bleu de chrome, bromocrésol pourpre, carmin indigo, cacotheline, calcéine, eosine extra, eriochromocyanine, jaune thiazol, noir d'eriochrome T, α-nitro β-naphtol, orange III, rouge methyl, rhodamine B, rhodizonate de potassium, thymolsulffonephtalein, rhodium chlorure, trihydroxy2,6,7phenyl9 isoxanthen, thorin, xylene orange tetranatrium salz, anthocyanes, phénolphtaléine, le benzopurpurin 4B, benzopurpurin B, alpha naphtyl red hydrochloride, litmus, Rouge de méthyle, Indicateur Mixte, Lacmoïd.

Dans une réalisation, au moins un des paramètres de l'indicateur est tel que l'indicateur soit adapté au produit, les paramètres étant compris dans le groupe suivant : la nature de la substance microbienne, la quantité de cette substance microbienne, à nature des nutriments, la quantité de ces nutriments, la nature des éléments nécessaire à la production d'acide par la substance microbienne et la quantité de ces éléments, la nature d'un texturant du milieu dans lequel se trouve la substance microbienne et la quantité de ce texturant, le pH de départ du milieu, les paramètres influant sur l'a_{w} du milieu, la nature du révélateur utilisé pour mesurer la baisse du pH, et la quantité de ce révélateur, les paramètres déterminant le pH auquel ce révélateur change d'état.

Dans une réalisation, l'indicateur est tel que les paramètres sont tels que le signal indiquant le changement d'état apparaisse au bout d'une durée prédéterminée lorsque le produit est conservé dans des conditions standards ou préconisées.

Dans une réalisation, le dispositif comprend un indicateur dans lequel les paramètres sont tels que le signal indiquant le changement d'état apparaît lorsque le produit n'est plus consommable ou utilisable.

Dans une réalisation, les nutriments incluent une source de carbone et une source d'azote.

La source de carbone est par exemple comprise parmi les sucres du groupe suivant : Glycérol, Erythritol, D-Arabinose, L-Arabinose, Ribose, D-Xylose, L-Xylose, Adonitol, β Methyl-xyloside, Galactose, D-Glucose, D-Fructose, D-Mannose, L-Sorbose, Rhamnosé, Dulcitol, Inositol, Mannitol, Sorbitol, α Methyl-D-mannoside, α Methyl-D-glucoside, N Acetyl glucosamine,

Amygdaline, Arbutine, Esculine, Salicine, Cellobiose, Maltose, Lactose, Melibiose, Saccharose, Trehalose, Inuline, Melezitose, D-Raffinose, Amidon, Glycogène, xylitol, βGentiobiose, D-Turanose, D-Lyxose, D-Tagatose, D-Fucose, L-Fucose, D-Arabitol, L-Arabitol, Gluconate, 2 ceto-gluconate, 5 ceto-gluconate.

Dans une réalisation, un texturant est compris parmi ceux du groupe suivant : agar, agarose, gélatine, xanthane, scléroglucane, Gum Guar.

Dans une réalisation, l'indicateur est tel qu'il est inactivé quand il n'est pas associé au produit et activé lorsqu'il est associé au produit.

Dans une réalisation, l'inactivation est réalisable à l'aide de moyens compris dans le groupe suivant : congélation, microencapsulation de la substance microbienne et/ou des nutriments, compartimentation.

Dans une réalisation, l'activation de l'indicateur est réalisable par une action physique choisie dans le groupe suivant : variation de pression, variation de température, variation de la longueur d'onde d'exposition à un rayonnement.

Dans une réalisation, la substance microbienne est congelable et décongelable.

Dans une réalisation, l'indicateur se présente sous la forme d'une étiquette, de préférence autocollante.

Dans une réalisation, l'indicateur comprend au moins une face dont une zone permet d'observer le signal produit par le révélateur.

Dans une réalisation, l'étiquette comprend plusieurs compartiments internes, par exemple des capsules, permettant de séparer un ou plusieurs constituants du composant des autres pendant une durée déterminée, les parois desdits compartiments pouvant être rompues par tout moyen adapté, par exemple par pression sur l'étiquette.

D'autres caractéristiques et avantages de la présente invention apparaîtront à la lecture de la description de différents modes de réalisation, la description étant faite avec référence aux dessins ci-annexés dans lesquels :
La Figure 1 illustre l'acidification du milieu par différentes souches à basses températures en fonction du temps ;
La Figure 2 illustre la croissance de la souche CRYO-CB002 à différentes températures ;
La Figure 3 illustre le taux de croissance de la souche CRYO-CB002 avec 10 ou 20 g/l de glucose, lactose, galactose, fructose ou du saccharose.
La Figure 4 illustre un exemple de cinétique d'acidification du milieu par une bactérie lactique ;
La Figure 5 illustre l'évolution de la force du gel en fonction de la concentration en gélatine et de la force de bloom ;
La Figure 6 illustre l'évolution du pH du milieu en fonction de différentes quantités inoculum de la souche CRYO-CB002 pour deux cas de conditions de conservation ;
La Figure 7 illustre l'évolution du pH en fonction de la concentration en gélatine ;
La Figure 8 illustre l'acidification du milieu par la souche CRYO-CB002 à 8°C avec différentes concentrations en glycérol ;
La Figure 9 illustre la vitesse d'acidification du milieu par la souche CRYO-CB002 en fonction du pH de départ pour deux températures ;
La Figure 10 illustre l'acidification du milieu par la souche CRYO-CB002 (inoculum 1.10^{E}4) en présence de 2% de glucose ou 2% de mannitol ;
La Figure 11 présente une photo montrant la précipitation de la caséine et le virage de l'indicateur mixte lors de l'acidification du milieu par des bactéries lactiques ;
La Figure 12 illustre une étiquette selon l'invention ;
La Figure 13 illustre un avantage de l'invention ;
La Figure 14 illustre un rouleau d'indicateurs selon l'invention et des moyens d'association des indicateurs aux produits ;
Les Figures 15 et 16 sont des graphiques illustrant des exemples d'utilisation du procédé selon l'invention ;

On rappelle ici la détermination de la DLC ou de la DLUO d'un produit périssable (norme AFNOR NF V-01-003). Pour un produit périssable, la durée de vie est déterminée en fonction des températures moyennes de conservation.

La norme AFNOR NF V-01-003, validée en février 2004, relative à l'hygiène et à la sécurité des produits alimentaires fournit les lignes directrices pour l'élaboration d'un protocole de test de vieillissement pour la validation de la durée de vie microbiologique de denrées périssables, réfrigérées. Elle décrit une méthode d'évaluation de la date limite de consommation (DLC) ou de la date limite d'utilisation optimale (DLUO) d'un produit microbiologiquement périssable.

La norme précise que la date limite de consommation ou d'utilisation optimale d'un produit périssable doit être déterminée en tenant compte du degré de maîtrise de la chaîne du froid chez les différents intervenants (fabricant, transporteur, distributeur et consommateur). Cette date limite est donc déterminée en fonction des conditions moyennes, généralement observées le long de la chaîne du froid durant la conservation d'un produit.

Cette norme définit trois cas de figure pour déterminer cette date limite en fonction du niveau de maîtrise estimé de la chaîne du froid.

Si la chaîne du froid est totalement maîtrisée, la date limite est déterminée pour un produit conservé pendant toute sa durée de vie à la température optimale (+4°C par exemple pour la plupart des produits alimentaires).

Si la chaîne du froid est partiellement maîtrisée la date limite est déterminée pour un produit conservé deux tiers du temps de sa durée de vie à la température optimale (par exemple +4°C) et un tiers du temps à la température raisonnable de rupture de la chaîne du froid (en général +8°C).

Si la chaîne du froid est insuffisamment maîtrisée, la date limite est déterminée pour un produit conservé un tiers du temps de sa durée de vie à la température optimale et deux tiers du temps à la température raisonnable de rupture de la chaîne du froid.

Ainsi la date limite de consommation ou d'utilisation optimale d'un produit est une valeur fixée a priori en fonction de données statistiques et de conditions de conservation présupposées, cette date étant figée et n'évoluant pas en fonction des conditions de conservation réelles du produit périssable. Le problème est que cette date limite est une valeur statique qui ne peut rendre compte de la durée de vie réelle du produit puisque son mode de détermination présuppose le niveau de maîtriser de la chaîne du froid exercée sur un produit par les différents intervenants (fabricant, transporteur, distributeur et consommateur). Cette date limite ne constitue donc qu'une indication théorique et statistique de l'état sanitaire du produit périssable après une période donnée.

Le procédé permet d'apporter une solution à ce problème et de réduire les risques sanitaires en donnant la date limite de consommation ou la date limite d'utilisation optimale de façon dynamique pour chaque produit individuellement en fonction des conditions de conservation auxquelles le produit aura effectivement été soumis.

L'indicateur peut être paramétré pour évoluer de façon identique au produit dans les mêmes conditions de conservation. Ainsi, la date limite de consommation ou la date limite d'utilisation optimale du produit est donnée par l'indicateur et évolue en fonction des conditions de conservation réelles du produit périssable.

Dans une première application, l'indicateur peut être paramétré pour changer d'état à la date limite de consommation ou d'utilisation d'un produit périssable telle quelle est déterminé selon la normé AFNOR NF-01-003. Si les conditions de conservation du produit sont moins bonnes que les conditions moyennes prévues par cette norme, l'indicateur signale que le produit n'est plus consommable avant la date initialement prévue ; si les conditions de conservation du produit sont meilleures (température de conservation optimale sans aucune rupture de chaîne du froid ou congélation du produit) que les conditions moyennes prévues, l'indicateur signale que le produit est toujours consommable au delà de la date initialement prévue.

Dans une deuxième application, en s'appuyant sur une étude de la dégradation microbiologique spécifique de chaque produit, l'indicateur peut être paramétré pour évoluer de façon identique au produit en fonction de ses conditions de conservation (temps et température) et ainsi se substituer totalement à la DLC ou la DLUO du produit telle qu'elle est déterminée selon les normes actuelles et telle que le consommateur la connaît aujourd'hui. Cela est rendu possible par un paramétrage de l'indicateur fondé sur la vitesse de dégradation microbiologique réelle du produit.

Il existe de nombreux produits qui sont périssables et sensibles à la dégradation ou à l'altération, microbiologique ou non, en fonction de leur durée et de leur température de conservation. Il s'agit, de préférence, de produits alimentaires frais ou surgelés, par exemple, vendus en magasin ou distribués par des sociétés de restauration (en liaison chaude ou froide), de médicaments (vaccins, collyres), de substances médicalement actives (sang), de produits chimiques thermosensibles (réactifs de diagnostic ou d'analyse, révélateurs photographiques), de produits cosmétiques, de fleurs coupées ou de produits consommables sensibles (vins, eaux minérales, cigares). Chacun de ces produits possède sa propre sensibilité et sa propre cinétique de dégradation en fonction de la température et de la durée de conservation, et donc sa propre date limite de consommation ou date limite d'utilisation optimale dans les conditions de conservation recommandées. Quelques exemples sont donnés dans le tableau suivant :

| PRODUIT | CONSERVATION | |
|---|---|---|
| | Température | Durée |
| Découpe de volaille | 0°C à +4°C | 7 jours |
| Rillettes | 0°C à +4°C | 29 jours |
| Lardons | 0°C à +4°C | 50 jours |
| Plats cuisinés liaison froide | 0°C à +3°C | 1 à 6 jours |
| Plats cuisinés liaison chaude | > à +63°C | Quelques heures |
| Sang | +2°C à +8°C | 42 jours |
| Vaccins | +2°C à +8°C | 2 à 6 mois |
| Eau minérale | +18°C à +20°C | 15 jours après ouverture |
| Chocolat | +8°C à +18°C | 12 mois |
| Antithrombotiques | +15° à +25°C | 24 mois |

L'indicateur selon l'invention est paramétrable afin de couvrir les différentes DLC ou DLUO correspondant aux produits périssables, de sorte que son changement d'état intervienne à la date prévue si le produit est conservé dans les conditions qui ont été choisies pour fixer cette date limite.

### L'INDICATEUR :

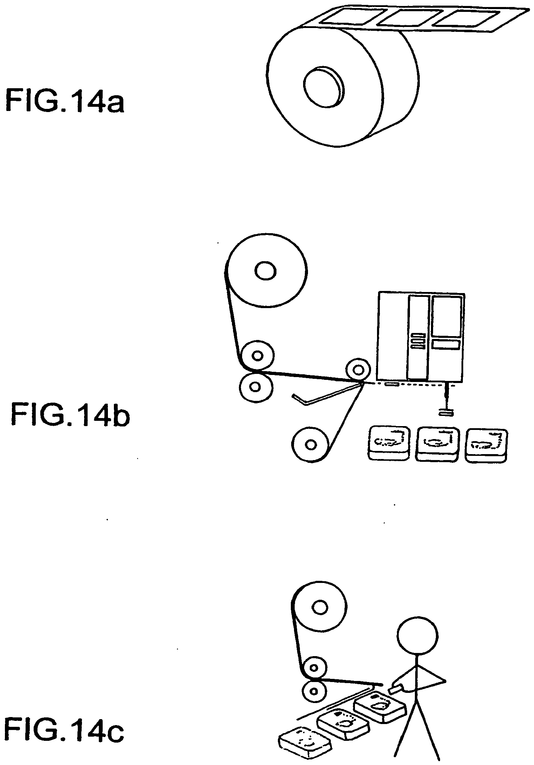

L'indicateur se présente sous la forme d'une étiquette transparente d'épaisseur réduite et de surface adaptée à l'utilisation et au produit sur lequel elle sera apposée. Selon une réalisation, l'étiquette est autocollante et sa surface est de la taille d'un code à barres afin d'être appliquée sur celui-ci comme décrit dans la demande de brevet antérieur WO03/025529. L'épaisseur de l'étiquette est préférentiellement suffisamment réduite possible pour être discrète et pour s'intégrer au produit (ou à l'emballage) sur lequel elle est apposée et ne pas gêner la lecture des informations qu'elle recouvre. Dans une réalisation, les étiquettes autocollantes sont délivrées en rouleaux, tel que représenté dans la figure 14-a, utilisables sur une chaîne de conditionnement automatique ou manuelle et sont déposées à la main, tel que représenté dans la figure 14-c, ou à l'aide d'une étiqueteuse industrielle, tel que représenté dans la figure 14-b. Selon cette réalisation, la fabrication et l'utilisation de l'indicateur sont effectuées à cadence élevée et à des coûts raisonnables afin de répondre aux besoins et contraintes de l'industrie et de la grande série.

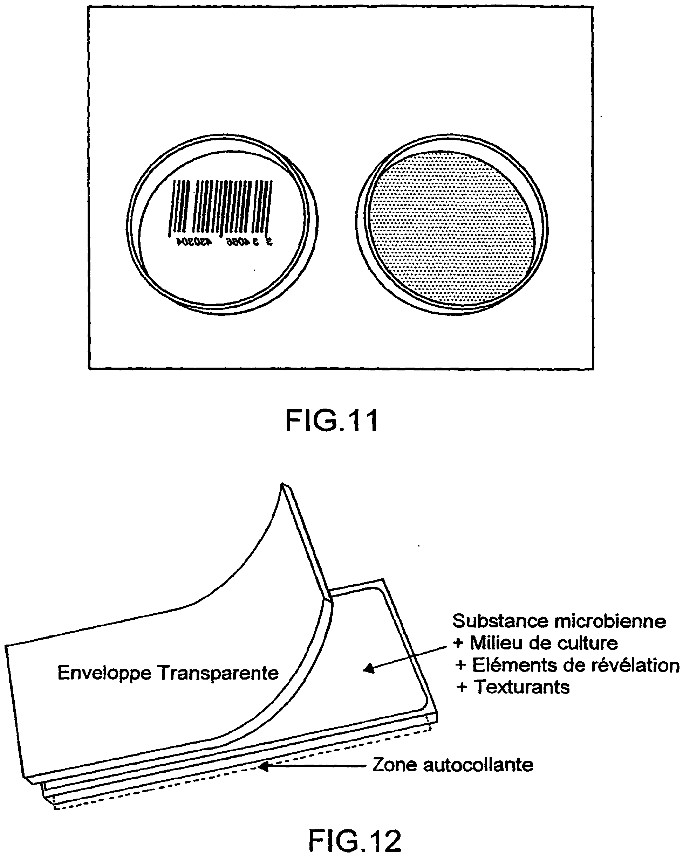

L'indicateur comprend donc une enveloppe transparente, facultativement autocollante, contenant une substance microbienne acidifiante, un milieu de culture, les éléments de révélation de l'acidification, et un ou plusieurs texturants, comme indiqué dans la Figure 12.

L'enveloppe transparente de l'indicateur est, par exemple, constituée d'au moins l'un des matériaux suivants : Polyéthylènetéréphtalate (PET), polyéthylène (PE) et polypropylène (PP).

### SUBSTANCE MICROBIENNE :

Pour la mise en oeuvre de l'invention, on fait appel à une substance microbienne faisant varier le pH du milieu dans lequel elle se trouve et cette substance microbienne est un microorganisme produisant un acide durant sa croissance et son développement ou via son activité métabolique. Elle est, par exemple, utilisée dans la composition de produits alimentaires (substance microbienne positive ou technique).

De plus, la substance microbienne doit présenter une activité acidifiante à des températures compatibles avec les températures et les durées de conservation des produits. Sa cinétique d'acidification doit également être compatible avec ces températures et ces durées de conservation et est en rapport avec les cinétiques de dégradation des produits périssables. C'est pourquoi l'indicateur selon l'invention comprend au moins une souche de bactéries choisie parmi la souche CRY0-CB001 et la souche CRY0-CB002 qui sont des bactéries lactiques. Cette dernière famille de bactéries est utilisée pour la transformation et la conservation de nombreux produits alimentaires : la plupart des bactéries lactiques ne présentent donc aucun risque à la consommation. La grande majorité de ces bactéries sont de grade alimentaire. Il s'agit d'une flore dite positive dont l'innocuité est anormalement assurée. Ainsi on peut les associer, sans risque, à des produits consommables.

Les bactéries lactiques produisent de l'acide (essentiellement de l'acide lactique) au cours de. leur croissance et de leur activité métabolique. Ces bactéries acidifient donc le milieu dans lequel elles se développent. Ces propriétés acidifiantes sont connues et utilisées dans de très nombreuses applications agroalimentaires.

Les bactéries lactiques se développent naturellement dans beaucoup de produits alimentaires périssables à des températures et à des vitesses de croissance proches des flores de dégradation et des flores pathogènes. L'utilisation de bactéries lactiques permet donc de refléter les cinétiques de contamination et de dégradation microbiologique des produits sur lesquels l'indicateur est apposé.

Dans la famille des bactéries lactiques, on a sélectionné les souches dont le comportement en fonction des températures et des vitesses de croissance, correspond le mieux aux cinétiques de développement des microorganismes dégradant les produits périssables. Il est important que les vitesses et les températures auxquelles les microorganismes se développent, ou possèdent une activité métabolique acidifiante, soient compatibles avec les température de conservation du produit périssable et compatibles avec les cinétiques de dégradation ou d'altération de ces produits.

Les produits périssables se conservent généralement à froid (entre 0°C et +4°C), mais sont susceptibles d'être exposés à des températures supérieures lors d'une rupture de la chaîne du froid. Des souches de bactéries lactiques psychrotrophes, tout en restant relativement mésophiles, sont sélectionnées pour l'indicateur. Il s'agit de souches capables de vivre, de se développer ou d'avoir une activité métabolique acidifiante à des température allant en général de 0°C à +45°C. Selon un mode préféré de réalisation, la croissance ou l'activité métabolique acidifiante doit être d'intensité croissante de 0°C à +37°C afin que la vitesse de croissance de ces souches (ou l'intensité de leur activité métabolique acidifiante) soit compatible avec la cinétique de dégradation du produit périssable.

Par définition, les bactéries mésophiles sont des bactéries qui peuvent vivre à des températures variant de +20°C à +45°C et dont le développement est optimal à +37°C. Les bactéries psychrotrophes sont des bactéries dont le développement est optimal à des températures variant de +20°C à +30°C mais qui peuvent vivre et se développer à 0°C**.** Les bactéries psychrophiles sont des bactéries qui peuvent vivre à des température variant de -5°C à +30°C et dont le développement est optimal à +15°C. Les bactéries cryophiles sont des bactéries qui se développent dans les milieux froids.

Pour arriver à l'invention, on a étudié les caractéristiques de croissance de 131 souches de bactéries lactiques par exemple parmi les genres Lactobacillus, Enterococcus, Carnobacterium, Leuconostoc et Weissella. Des souches capables de croître à basses températures (à partir de 0°C et au minimum +4°C et +8°C). Ensuite, on a mesuré les valeurs cardinales (en fonction de la températures, du pH et de l'a_{w}) des souches donnant les meilleurs résultats.

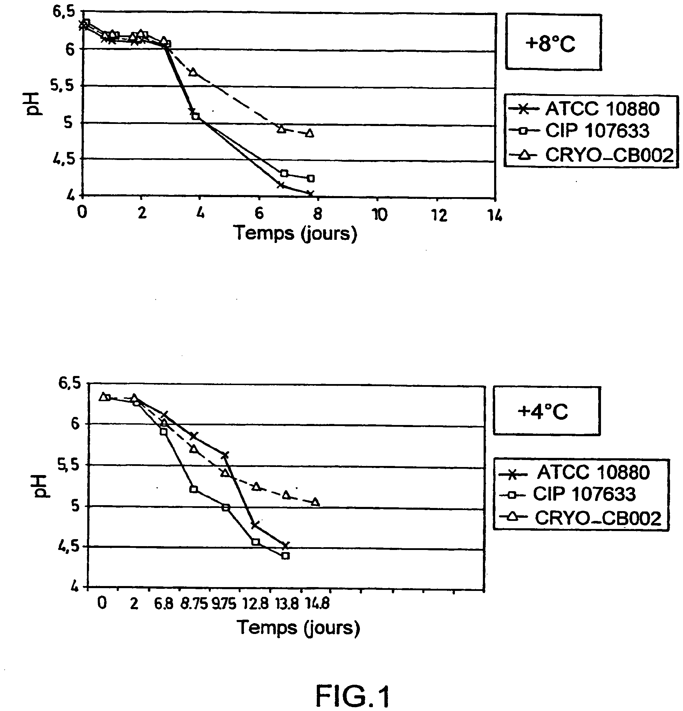

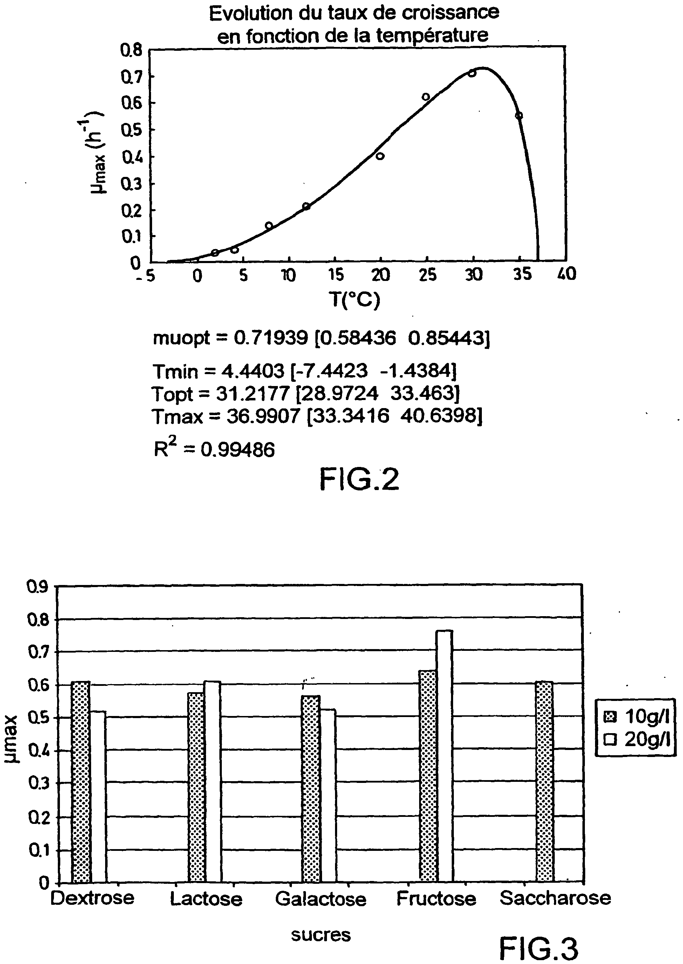

Parmi ces souches, selon l'invention deux souches de l'espèce Carnobacterium piscicola, la souche CRYO-CB001 et la souche CRYO-CB002 isolées par les inventeurs et enregistrées respectivement sous les numéros CNCM 1-3297 et CNCM 1-3298 dans la Collection Nationale de Culture de Microorganismes de l'Institut Pasteur ont été retenues. Ces souches peuvent être utilisées en raison de leurs activités métaboliques acidifiantes à basse température. La Figure 1 représente l'acidification du milieu à +4°C et +8°C pour l'indicateurs paramétré comme suit : Glucose 20 g/l - Tryptone 10 g/l - Extrait de Levure 5 g/l-Tween 80 1,08 g/l- Phosphate dipotassique anhydre 2,6 g/l-Sulfate de magnésium anhydre 0,222 g/l - Sulfate de manganèse anhydre 0,055 g/l - pH 6,2- Inoculum 10^{E}4 CFU/ml. Par exemple, la souche CRYO-CB002 est capable de croître à des température, comprises entre -4°C et +37°C, ainsi que représenté dans la Figure 2, tout en résistant à des températures inférieures à - 80°C et supérieures à +47°C dans l'indicateur. Ceci permet de couvrir la majeure partie des températures qui peuvent être rencontrées durant la conservation d'un produit périssable et notamment un produit alimentaire frais.

La cinétique d'acidification du milieu contenu dans l'indicateur, par la substance microbienne, dépend du métabolisme de cette substance mais dépend aussi de la quantité de substance microbienne introduite dans l'indicateur. De façon générale, plus la quantité de substance microbienne introduite dans l'indicateur est importante et plus l'acidification du milieu qu'il contient sera rapide (pour une température donnée). Inversement, moins la quantité de substance microbienne introduite dans l'indicateur est importante et moins l'acidification du milieu sera rapide. Ceci est vrai pour une formulation donnée du milieu.

Cela signifie donc que l'évolution de l'indicateur dépend du type mais aussi de la quantité de substance microbienne qu'il contient. Ainsi, en fonction des réalisations, on pourra introduire dans l'indicateur plus ou moins de substance microbienne de façon à obtenir une acidification du milieu, et donc un signal de changement d'état, plus ou moins rapide.

### LE MILIEU DE CROSSANCE

L'indicateur contient une substance microbienne acidifiante. Cette substance microbienne doit donc se trouver dans un milieu permettant son développement, sa croissance ou son métabolisme acidifiant.

Par exemple, lorsque des bactéries lactiques sont utilisées, l'indicateur doit contenir un milieu de culture permettant la survie et la croissance des bactéries mais aussi permettant la production d'acide par le métabolisme de ces bactéries.

De façon générale, pour un bon développement bactérien, le milieu de culture doit contenir, une source de carbone (en général un sucre), une source d'azote, (en général d'origine protéique)., des sels minéraux, diverses vitamines, oligoéléments ou acides gras essentiels.

Pour leur croissance, les souches de bactéries lactiques peuvent être cultivées sur un milieu contenant différentes sources de carbone. Les sucres suivants peuvent être utilisés pour la croissance des souches CRYO-CB001 et CRYO-CB002 : Glycérol, Ribose, Galactose, D-Glucose, D-Fructose, D-Mannose, Mannitol, a Methyl-D-glucoside, N Acetyl glucosamine, Amygdaline, Arbutine, Esculine, Salicine, Cellobiose, Maltose, Lactose, Saccharose, Trehalose et β Gentiobiose.

Le sucre contenu dans le milieu, par exemple le glucose, sert aussi à la production d'acide lactique par les bactéries. Il est donc aussi nécessaire à l'activité acidifiante de la bactérie. Cela signifie que l'indicateur doit contenir un sucre utilisable par la bactérie pour son métabolisme acidifiant, par exemple le glucose ou le fructose.

La nature du sucre utilisables par la bactérie, par exemple le glucose, le fructose, le lactose, le galactose ou le saccharose, modifie la vitesse de croissance des bactéries, par exemple de la souche CRYO-CB002, tel que présenté sur la Figure 3, et la cinétique d'acidification du milieu.

Dans un mode préféré de réalisation, le glucose est utilisé pour la croissance et l'activité acidifiante avec les souches CRYO-CB002 et CRYO-CB001.

Une source d'azote est également nécessaire à la croissance des bactéries lactiques. Différentes sources, par exemple la tryptone, la peptone ou tout composé d'origine protéique sont utilisables. Le type et la quantité de la source d'azote contenue dans le milieu influent sur le développement bactérien.

La tryptone et la peptone peuvent être utilisées au sein de l'indicateur à des concentrations de 1% ou 2% en masse (w/w), seules ou associées.

Selon un mode de réalisation préféré avec la souche CRYO-CB002 un milieu contenant 1% de peptone a été validé et selon un autre mode préféré de réalisation avec la même souche, un milieu contenant 1% de tryptone a été validé.

Comme recommandé pour le milieu MRS (de Man, Rogosa, Sharpe), de l'extrait de levure est ajouté au milieu, de même des sels (par exemple NaCl, K₂HPO₄ et MgSO₄), nécessaires ou favorisant la croissance ou au métabolisme des bactéries lactiques peuvent être ajoutés.

Par exemple, un milieu utilisé est composé de :

| | |
|---|---|
| Tryptone | 1% |
| Extrait de Levure | 0,5% |
| Tween 80 | 0,108% |
| Phosphate dipotassique anhydre | 0,26% |
| Sulfate de magnésium anhydre | 0,0222% |
| Sulfate de manganèse anhydre | 0,0055% |
| Glucose | 2% |

La composition en nutriments du milieu influe donc sur la vitesse de croissance des souches, leur activité métabolique acidifiante et donc le paramétrage de l'indicateur.

Enfin, le pH du milieu contenant la substance microbienne acidifiante est important. En effet, il faut que le pH du milieu soit compatible avec la croissance et l'activité métabolique de la substance microbienne, par exemple un pH entre 9 et 4 pour des bactéries lactiques. Par exemple, pour la souche CRYO-CB002, le pH optimum de croissance est de 7,1. Pour qu'il puisse y avoir acidification du milieu par la substance microbienne, le pH de départ de ce milieu doit être suffisamment élevé (en général supérieur à 6 pour des bactéries lactiques) tout en étant compatible avec le développement et le métabolisme acidifiant de la substance microbienne.

Comme la cinétique d'acidification a une influence sur le paramétrage de l'indicateur, le pH de départ du milieu contenant la substance microbienne a également une influence sur ce paramétrage.

### LA REVELATION DE L'ACIDIFICATION:

Dans l'indicateur selon l'invention on simule la dégradation d'un produit périssable grâce à une substance microbienne. Le développement de la substance microbienne et son métabolisme acidifiant sont visualisés par le suivi de L'acidification du milieu contenu dans l'indicateur. L'acidification du milieu peut être visualisée à l'aide de plusieurs révélateurs.

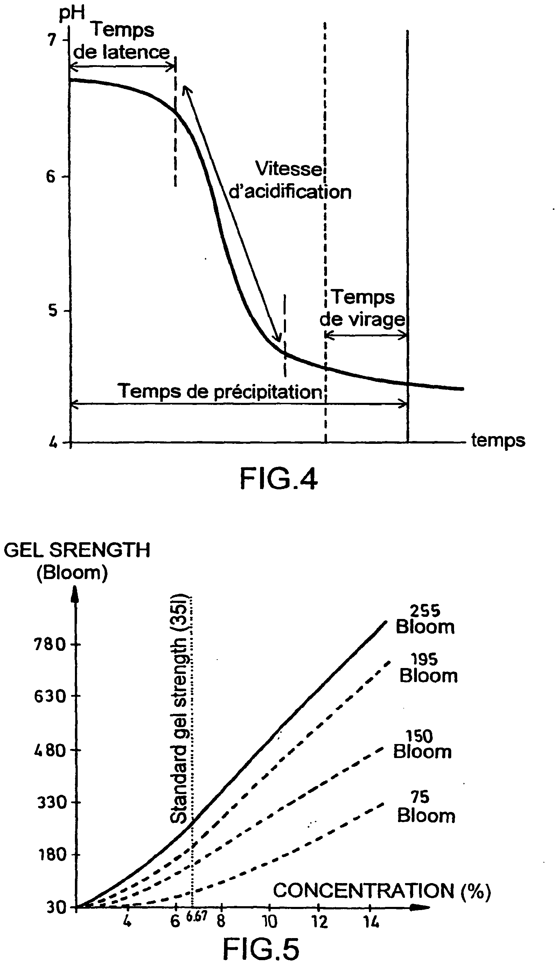

La fin de la DLC ou de la DLUO d'un produit est traduite par l'indicateur lorsque celui-ci change d'état. L'indicateur change d'état lorsque le pH du milieu qu'il contient passe en dessous d'une valeur déterminée, dont un exemple est illustré sur la Figure 4. Le passage du pH en dessous d'une valeur déterminée est visualisé par un changement d'état d'un ou plusieurs révélateurs contenus dans l'indicateur.

Des indicateurs colorés sont utilisés afin de visualiser l'acidification du milieu. Les indicateurs colorés sont des acides ou des bases faibles dont les formes acide et basiques ont des couleurs différentes. La couleur des indicateurs change donc lorsque le pH du milieu qui les contient passe en dessous, ou au dessus, du pKi de l'indicateur.

Ainsi, la couleur de l'indicateur peut changer à un pH déterminé si l'indicateur coloré qu'il contient possède un pKi égal au pH en question. Un des intérêts d'utiliser des indicateurs colorés est que le signal par changement de couleur peut être interprété à l'oeil nu. De plus, le changement de couleur intervient de façon franche et brutale ce qui évite la confusion due à une interprétation subjective.

De nombreux indicateurs peuvent être utilisés pour le procédé, par exemple, le rouge de méthyle, l'Indicateur Mixte, le Lacmoïd, les anthocyanes, la phénolphtaléine, le 2,4 dinitrophénol, l'éthyl orange, le benzopurpurin 4B, le benzopurpurin B, l'alpha naphtyl red hydrochloride, le litmus, l'acide aurine, le dithiozone, le bleu de chrome, le bromocrésol pourpre, le carlin indigo, le cacotheline, la calcéine, l'eosine extra, l'eriochromocyanine, le jaune thiazol, le noir d'eriochrome T, l'a-nitro β-naphtol, l'orange III, le rouge methyl, la rhodamine B, le rhodizonate de potassium, le thymolsulffonephtalein, le rhodium chlorure, le trihydroxy2,6,7phenyl9 isoxanthen, le thorin, le xylene orange tetranatrium salz.

Les indicateurs colorés suivants peuvent aussi être utilisés au sein de l'indicateur: Alizarine, Alizarine Sodium Sulfonate, Alzarine Red ,Benzopurpurin, Benzoyl Auramine G, Benzoylethylauramine, Bleu De Résorcine, Bleu De Bromochlorophénol, Bromocresol Green, Bromophenol Blue, Carmine Acid, 2,4-Dinitrophenol, 4-(4-Dimethylamino-1-Naphtylazo)-3-Methoxybenzenesulfonic Acid, α-dinitrophénol, β-dinitrophénol, γ-dinitrophénol, Disodium4,4- Bis(O-Tolytriazeno)-2,2'-Stilbenedisulfonate, Disodium4, 4- Bis (P-Dimethylaminophenylazo)-2,2'-Stilbenedisulfonate, 4-(P-Ethoxyphenylazo) M-Phenylene-Diamine Monchydrochloride, Ethyl Red, Ethyl Orange, Hexamethoxy Red, Aydroquinolsulphonaphtalein, Lacmoid, Iodeosin, Lasmoid, N, Ndimethyl-P-(M-Tolylazo) Aniline, 4'-Methoxy-2.4-Diaminoazobenzene, Methyl Red, Methyl Red-Alphazurin, α-Naphtylamine, α-Naphtylaminoazo-Benzène, Naphtyl Red, Oxime Blue, 4'-Oxy-3'-Methyl-2.4-Diaminoazobenzene, 4'-Oxy-2.4diaminoazobenzène, 4-Phenylazo-1-Naphtylamine, Parafuchsine-Hexa-Acetic Acid, Paranitrophénol, P-Ethoxychrysoidine, P-Sulfo-O-Methoxybenzeneazodimethyl- αNaphtylamine, Rouge Congo, Red Violet, Resazurin, Rouge Naphtyl, Tetrabromocresol, Tétraiodophenolsulfophthalein.

Selon des réalisations préférées, les indicateurs suivants sont utilisés : les anthocyanes, la phénolphtaléine, le 2,4 dinitrophénol, l'éthyl orange, le benzopurpurin 4B, le benzopurpurin B, l'alpha naphtyl red hydrochloride, le litmus, le Rouge de méthyle, l'Indicateur Mixte, le Lacmoïd.

Selon un mode de réalisation préféré particulier, l'indicateur mixte est utilisé car il présente des qualités esthétiques et techniques intéressantes. Son virage coloré s'effectue à un pH proche du pH de précipitation de la caséine et ses couleurs, vert avant virage et bleu après virage, correspondent à un code couleur utilisable pour le grand public, notamment en terme de marketing.

Toutefois, d'autres indicateurs colorés peuvent être utilisés en fonction des besoins, notamment marketing, liés au produit périssable et à son emballage.

Un autre moyen pour visualiser que la date limite de consommation ou d'utilisation optimale d'un produit périssable est dépassée et de rendre opaque l'indicateur qui au départ est transparent.

On a décrit dans la demande de brevet antérieure WO03/025529 un procédé pour contrôler systématiquement un produit dont la conservation dépend de la chaîne de froid. La péremption du produit peut être détectée systématiquement par l'occultation d'un code à barres sur lequel l'indicateur/étiquette est apposé.

Dans un mode de réalisation, l'étiquette occulte un message sur lequel elle est apposée. Ce message pouvant indiquer que le produit est consommable ou utilisable tant qu'il est lisible.

La caséine peut être utilisée pour opacifier l'indicateur lorsqu'il y a acidification du milieu.

La caséine est l'une des principales protéines du lait. Plus de la moitié de ses acides aminés portent des groupements ionisables libres. Dans le lait, la caséine se trouve dans un complexe salin à l'état micellaire. Une chute du pH provoque une précipitation de la caséine. Elle précipite totalement vers un pH de 4.7 dans le lait. Si l'acidification se développe progressivement dans le milieu, du fait d'une fermentation lactique, il se forme un coagulum homogène, comme dans le yaourt. Ainsi, la caséine soluble dans le lait précipite en présence d'acide lactique et se retrouve sous une forme insoluble:

*caséinate-Ca + 2 CH₃-cHOH-COOH* => *caséine* + *(CH₃-CHOH-COO)₂ Ca*

### (Précipitation de la caséine par l'acide lactique)

Cette réaction est très utilisée en agroalimentaire, lors du caillage du lait pour la formation du fromage frais puis du fromage salé et affiné.

La caséine se prête parfaitement à la réalisation de l'invention puisqu'elle peut être en solution transparente à des pH basiques, neutres (pH 7) ou légèrement acides (supérieurs à son point isoélectrique) et qu'elle peut former un précipité opaque à des pH bas (inférieurs à son point isoélectrique). Le pH de précipitation de la caséine varie en fonction de la force ionique du milieu et de la température. Au sein de l'indicateur, en fonction des concentrations salines choisies, la caséine précipite à des pH compris entre 5,7 et 5.

De plus, la caséine est un produit peu cher, de grade alimentaire qui est utilisé dans de nombreuses applications de l'industrie agroalimentaire.

La caséine peut être extraite du lait par précipitation acide puis neutralisée par de la soude (NaOH), de la potasse (KOH) ou de la chaux (CaOH). On obtient alors des caséinates de sodium, de potassium ou de calcium respectivement. La caséine peut de nouveau être remise en solution par dissolution de ces caséinates.

La caséine peut aussi être extraite du lait par microfiltration ou ultrafiltration tangentielle. Grâce à ce procédé, on récupère une caséine native plus ou moins contaminée par d'autres protéines solubles du lait.

Pour l'indicateur, des caséinates de sodium (Na+), de calcium (Ca++), de potassium (K+) ou des caséines natives (non précipitées) de différentes puretés peuvent être utilisés.

De préférence, au sein de l'indicateur, des concentrations en caséinate de sodium et caséinate de potassium de 5%, 6%, 7%, 8%, 9% et 10% sont introduites. Les caséines natives et le caséinate de calcium ont été utilisés à des concentrations de 0,5%, 0,75% et 1%. A ces concentrations, les différentes caséines sont solubles sous forme transparente dans le milieu. Elles permettent donc la lecture du code à barres au travers de l'indicateur.

L'intensité du précipité, et donc l'intensité de l'opacification de l'indicateur, dépendent de la concentration en caséine. Plus la concentration en caséine dans l'indicateur est élevée et plus l'intensité de l'opacification est importante. La concentration de la caséine dans l'indicateur doit être suffisante pour permettre l'opacification de l'indicateur après précipitation et empêcher la lecture du code à barres ou du message placé sous l'indicateur.

De préférence, en raison de la forte intensité du précipité obtenu, le caséinate de sodium est utilisé au sein de l'indicateur. De préférence, ce caséinate est utilisé à des concentrations comprises entre 8% et 10%.

Les deux types de révélateurs (indicateur coloré et caséine), peuvent être utilisés ensemble ou séparément.

S'ils sont utilisés ensemble, l'indicateur coloré peut être choisi pour changer de couleur au même pH que celui auquel la caséine précipite. Dans ce cas le signal de l'un renforce, ou appuie, le signal de l'autre. Par exemple, l'indicateur coloré vient renforcer l'indication, ou ajouter à la précipitation de la caséine un signal adapté au packaging ou aux goûts du consommateur.

Les deux types des révélateurs peuvent aussi être utilisés ensemble mais en choisissant un indicateur coloré qui change de couleur à un pH différent du pH de précipitation de la caséine, ceci pour donner deux messages successifs sur la possibilité d'utiliser ou non un produit périssable.

Par exemple, la précipitation de la caséine peut arriver en premier (à pH 5,2) et, en occultant le code à barres, être un signal indiquant que le produit ne peut plus être vendu (Date limite de Vente) ou doit être consommé rapidement. Le virage de l'indicateur coloré arrive ensuite (à pH 5 ou 4,5) et indique ainsi que le produit ne doit absolument plus être consommé. Pour cela on choisit un indicateur coloré dont le pH de virage est inférieur au pH de précipitation de la caséine et on peut quantifier et paramétrer cette différence.

L'inverse peut aussi être réalisé c'est-à-dire l'utilisation d'un indicateur coloré qui vire avant que la caséine ne précipite, c'est-à-dire un indicateur qui change de couleur à un pH plus élevé que le pH de précipitation de la caséine.

La Figure 11 montre une photo d'un exemple de précipitation de la caséine et de virage de l'indicateur mixte suite à une acidification du milieu selon l'invention indicateur comprend, dans cet exemple, des bactéries lactiques).

### LES TEXTURANTS:

L'indicateur est avantageusement présenté sous la forme d'une étiquette transparente de dimensions réduites, l'ensemble du contenant et du contenu étant transparent ou translucide. L'indicateur est relativement souple et homogène afin de s'adapter à tous les produits périssables et d'être appliqué sur tous les emballages dont on veut contrôler la dégradation.

Un texturant peut être ajouté au milieu afin de lui donner l'aspect désiré. Le texturant choisi doit permettre le développement de la substance microbienne dans l'indicateur, l'acidification du milieu et la révélation de cette acidification selon le mode choisi. De plus, le texturant ajouté doit être transparent et, si possible, incolore en solution dans le milieu afin de ne pas interférer avec le mode de détection de l'acidification, en particulier si le procédé d'occultation d'un code à barres est utilisé. Enfin, le texturant doit être, de préférence, de grade alimentaire pour faciliter une utilisation en industrie agroalimentaire de l'indicateur.

Selon un mode préféré de réalisation, le texturant utilisé est un épaississant ou un gélifiant.

Les épaississants augmentent la viscosité d'une solution. Ils ralentissent ou empêchent, selon leur concentration et la température, l'écoulement de la solution qui les contient. Ils sont utiles dans l'indicateur pour garder le milieu dans un état homogène et uniforme.

Les gélifiants solidifient la solution qui les contient en formant un réseau macromoléculaire permettant la formation d'un gel plus ou moins souple et élastique selon le type de gélifiant, sa concentration et la température. Ils sont classiquement utilisés en microbiologie lorsqu'un support solide est recherché pour la croissance bactérienne. La gélification de l'indicateur peut être utile pour permettre de garder le milieu dans un état homogène et uniforme et prévenir des écoulements si l'emballage de l'indicateur est percé.

Parmi les différents texturants qui peuvent être utilisés dans l'indicateur, figurent l'agar ou l'agarose (à des concentrations de 0,5 à 1,6%), différentes gélatines de différentes origines animales (porc, volaille, boeuf) et de différents tissus (peau, os), du xanthane, du scléroglucane, du Gum Guar. Le tableau suivant regroupe divers texturants susceptibles d'être également utilisés :

| **ORIGINE** | **TYPE** | **FONCTIONNALITES** |
|---|---|---|
| Extraits d'algues | Alginate | Epaississant Gélifiant |
| | Agarose | Gélifiant |
| Extraits de graines | Guar | Epaississant |
| | Caroube | Epaississant |
| Extraits de sous produits végétaux | Pectine | Gélifiant |
| Microorganismes, fermentation | Xanthane | Epaississant |
| | Gellane | Gélifiant |
| | Scléroglucane | Epaississant |
| Animale | Gélatine | Gélifiant |

De préférence, on utilisera de l'agarose ou de l'agar, de la gélatine ou du scléroglucane.

De façon générale, plus la concentration en texturant est élevée et plus la substance microbienne contenue dans l'indicateur se développe lentement et plus l'acidification du milieu de l'indicateur est lente. Ainsi la concentration en texturant influe sur le paramétrage de l'indicateur.

Parmi les texturants utilisables, la gélatine présente l'avantage d'être utilisée dans la fabrication de nombreux produits alimentaires et d'être de grade alimentaire. Son coût est relativement peu élevé ce qui permet une utilisation très répandue. Il en existe de différentes origines (bovine, porcine, volaille, poisson). En fonction de leur origine et de leur mode de préparation (extraction acide ou basique) et de la température d'extraction, les gélatines peuvent présenter différentes caractéristiques en terme de pouvoir gélifiant. Cette caractéristique est mesurée par la force de bloom. Plus la force de bloom d'une gélatine est élevée et plus le gel formé sera fort à une concentration donnée. Cela est illustré dans la Figure 5 (Chene C, 2000, La gélatine - Aspect théoriques ; Journal de l'ADRIANOR - Agro-Jonction N°24). En fonction du type de gélatine choisie et de sa force de bloom, l'aspect de l'indicateur pourra donc être modifié. Ainsi, une gélatine avec une force de bloom de 310, donnera un gel beaucoup plus solide qu'une gélatine avec une force de bloom de 200 à la même concentration et à la même température.

Des gélatines 150, 250 et 310 de porc, 150, 250 et 280 de boeuf, P200 de volaille sont notamment utilisables dans l'indicateur à des concentrations de 0,125% à 20%.

Plus le gel formé est fort, plus la substance microbienne se développe lentement et donc, plus l'acidification du milieu est lente. Ainsi, plus la gélatine possède une force de bloom élevée, plus l'acidification du milieu est lente.

De même, plus la gélatine (quelle que soit sa force de bloom) est concentrée, plus le développement microbien est lent et plus l'acidification du milieu est lente.

La force de bloom d'une gélatine et la concentration à laquelle cette gélatine est utilisée influent donc sur le paramétrage de l'indicateur. Ainsi, le type et la concentration du texturant utilisé permettent de faire varier l'aspect de l'indicateur mais aussi la vitesse d'acidification du milieu. On peut donc utiliser le texturant pour paramétrer l'indicateur en fonction du produit périssable à contrôler.

### ACTIVATION :

L'indicateur contient une substance microbienne qui se développe et acidifie le milieu plus ou moins rapidement en fonction de la température de conservation. L'indicateur se présente sous la forme d'une étiquette à poser sur un produit périssable. L'indicateur est paramétré pour suivre la dégradation du produit auquel il est associé. Il doit servir de Date limite de consommation ou d'utilisation optimale dynamique. Pour cela, le début du processus d'acidification doit correspondre au début de la DLC ou DLUO du produit périssable. Par commodité et pour des raisons économiques, la fabrication de l'indicateur est découplé, dans le temps et dans l'espace, de la fabrication et ou du conditionnement du produit périssable auquel il est destiné. Pour cela, le processus de développement microbien et l'acidification du milieu doivent pouvoir être déclenchés de façon indépendante de la fabrication.

Deux types de procédés peuvent être utilisés pour découpler la fabrication et l'activation de l'indicateur.

La congélation est un procédé d'inactivation réversible du développement microbien utilisable pour stopper la croissance et le métabolisme des bactéries contenues dans l'indicateur après la fabrication. Une fois l'indicateur assemblé (le milieu contenant la substance microbienne placé à l'intérieur de l'étiquette), l'indicateur est congelé à des températures négatives (températures auxquelles il n'y a normalement plus d'eau disponible à l'état liquide), par exemple, de -18°C à -80°C. Des températures de congélation inférieures à -18°C peuvent être utilisées. Cette température correspondant aux normes de congélation en vigueur en agroalimentaire et en usage domestique, elle est donc utilisée préférentiellement.

Une fois congelé, l'indicateur selon l'invention reste stable pendant plusieurs mois. Aucun développement bactérien et aucune acidification du milieu n'est observé. L'indicateur peut être stocké et transporté du lieu de fabrication au lieu d'utilisation sous cette forme congelée.

Les bactéries lactiques sélectionnées pour l'indicateur selon l'invention supportent la congélation au sein de l'indicateur et cette congélation n'affecte pas la capacité de développement des bactéries dès que celles-ci sont exposées à des températures compatibles avec leur croissance. Aussi, le système de révélation de l'indicateur n'est pas altéré par la congélation. La congélation laisse donc l'indicateur intact vis-à-vis de son principe actif. Il s'agit bien d'un procédé d'inactivation totalement réversible.

L'activation de l'indicateur se fait lorsque cet indicateur est décongelé en le plaçant à une température égale ou supérieure à la température à laquelle on peut trouver de l'eau à l'état liquide, en général à 0°C. Cela permet de nouveau le développement microbien et donc l'acidification du milieu. Le processus d'acidification du milieu et de révélation de cette acidification se déroule alors de façon identique au processus décrit précédemment.

L'autre technique utilisable au sein du procédé est un système d'activation conditionnelle de l'indicateur. Dans ce système d'activation conditionnelle, l'indicateur fabriqué (substance microbienne dans un milieu à l'intérieur d'une étiquette) n'est pas actif directement. L'activation de l'indicateur selon ce système doit être réalisée séparément de la fabrication par un procédé physique simple. Pour fabriquer un indicateur non actif et activable conditionnellement, la substance microbienne doit être isolée des nutriments (et réciproquement) pour empêcher tout développement de la substance microbienne ou son métabolisme acidifiant.

Cette séparation entre la substance microbienne et les nutriments peut être effectuée par incorporation au sein de l'indicateur de bactéries (ou bien de nutriments) encapsulés de manière étanche. Cela nécessite la fabrication préalable de capsules, ou billes, contenant la substance microbienne (ou les nutriments). Ces capsules doivent être relativement petites, étanches et stables dans le milieu contenu au sein de l'indicateur. Selon la taille des capsules, on parlera de microencapsulation (diamètre moyen des capsules inférieur à 1 mm) ou macroencapsulation (diamètre moyen des capsules supérieur à 1 mm). La substance microbienne isolée du reste du milieu par la capsule ne peut alors pas se développer ni acidifier le milieu. L'indicateur est donc inactif. L'indicateur ainsi fabriqué reste inactif et stable dans le temps, y compris à température ambiante si les capsules sont elles mêmes stables à température ambiante.

Pour activer l'indicateur, il faut libérer la substance microbienne (ou les nutriments) dans le milieu. Pour cela, il faut briser les capsules, si possible par un procédé physique simple (pression, ultra violet (UV), choc de température). La substance microbienne est alors libérée dans le milieu et peut se développer de nouveau. Réciproquement, si les capsules contiennent les nutriments, une fois ces capsules brisées, les nutriments sont disponibles pour le développement de la substance microbienne et l'acidification du milieu dans l'indicateur.

L'activation de l'indicateur est bien conditionnelle puisqu'elle survient lorsque les capsules sont brisées et lorsque la substance microbienne (ou les nutriments) est libérée. Grâce à cette technique, l'indicateur peut être produit en grande série, stocké et transporté à température ambiante sur le lieu de fabrication ou de conditionnement du produit périssable. L'indicateur peut alors être activé à la demande.

Il existe plusieurs techniques de micro ou macroencapsulation qui peuvent être utilisées. La coacervation complexe, l'encapsulation par atomisation d'émulsions inverses ou l'encapsulation par précipitation de l'alginate de sodium sont, par exemple, utilisées. Ces techniques peuvent être adaptées aux besoins spécifiques de l'invention : faible taille des capsules, étanchéité et stabilité totale en milieu aqueux, rupture par un procédé physique simple compatible avec les contraintes industrielles et microbiologiques.

Une autre façon d'envisager la séparation entre la substance microbienne et les nutriments est de fabriquer un indicateur constitué de deux compartiments différents. Un compartiment contient la substance microbienne dans un milieu sans nutriments et l'autre compartiment contient les nutriments isolés des bactéries. Les deux compartiments peuvent être séparés par une barrière transparente étanche, cette barrière pouvant être détruite ou interrompue par un procédé physique simple. Par exemple, si la barrière est un film de fabrication ou d'assemblage plus fragile que l'étiquette contenant l'indicateur, une pression de force calibrée peut rompre le film et mettre en contact les nutriments et les bactéries.

Dans le cas d'un système d'activation conditionnelle (encapsulation ou compartimentation) les bactéries doivent survivre et rester stables, y compris à température ambiante, dans un milieu en absence de nutriments. Par exemple, des bactéries lyophilisées peuvent être utilisées, ou bien des bactéries dans un milieu sans nutriments et ne contenant que des sels.

### PARAMETRAGE :

L'indicateur simule le développement des bactéries susceptibles de contaminer ou d'altérer le produit sur lequel il est apposé.

Dans une application, l'indicateur est paramétré en fonction de la DLC ou DLUO définie par l'industriel, en général selon la nome AFNOR NF V-01-003 : si le produit est conservé dans les conditions de températures qui sont celles utilisée pour définir cette date limite, l'indicateur change d'état à la date limite prédéfinie ; si le produit est conservé à des températures optimales (les moins favorables à la dégradation du produit et/ou au développement microbien) par rapport à celles présupposées par la norme, l'indicateur change d'état après la date limite théorique ; si le produit est conservé à des températures moins bonnes que celles présupposées par la norme (températures favorisant la dégradation du produit et/ou le développement microbien) l'indicateur change d'état avant la date limite théorique.

Dans une autre application, l'indicateur est paramétré pour refléter la dégradation, microbiologique ou non, du produit périssable. Ce paramétrage fait suite, par exemple, à une étude de microbiologie prévisionnelle spécifique à chaque produit ou à une étude de l'évolution des propriétés de stabilité du produit. Le changement d'état intervient lorsque les conditions de conservation (temps et température) du produit périssable entraînent un niveau d'altération et de dégradation dudit produit incompatible avec sa consommation ou son utilisation, et ceci indépendamment de la notion même de date limite.

L'invention décrit un indicateur qui constitue une date limite de consommation, ou d'utilisation optimale, dynamique d'un produit périssable.

La vitesse de croissance de la substance microbienne et la vitesse d'acidification du milieu par ladite substance microbienne doivent être adaptées, pour refléter la dégradation du produit périssable en fonction du temps et de la température de conservation. Cette adaptation est effectuée par le paramétrage de l'indicateur.

Ce paramétrage peut
(i) soit se baser sur la DLC ou la DLUO, du produit périssable, déterminée selon la norme AFNOR NF V-01-003; l'indicateur apporte alors une dimension dynamique par rapport à cette date limite prédéfinie en signalant que le produit n'est plus consommable avant cette date ou à l'inverse que le produit est consommable au-delà de cette date, en fonction de ses conditions réelles de conservation ;
(ii) soit se baser sur une étude de dégradation microbiologique réelle du produit et, parce que l'indicateur utilise des bactéries dont le comportement reflète le comportement les flores de dégradation, être le témoin exclusif du niveau de dégradation du produit périssable.

Dans tous les cas, l'indicateur doit être paramétré pour évoluer de façon à refléter le niveau de dégradation du produit en fonction de ses conditions (temps et température) de conservation.

Le paramétrage consiste à déterminer le moment auquel l'indicateur doit changer d'état, en fonction de la température et de la durée de conservation. C'est-à-dire le moment auquel le ou les révélateurs changent d'état au sein de l'indicateur.

Le paramétrage du couple révélateur/pH est donc essentiel. Ainsi, nous avons vu que le choix de l'indicateur coloré en fonction de son pH de virage est déterminant.

Pour un révélateur déterminé (par exemple l'Indicateur Mixte et/ou le caséinate de sodium), et dans des conditions de force ionique déterminée (composition saline du milieu permettant le développement de la substance microbienne) le contrôle du moment où le pH du milieu passe en dessous d'un seuil déterminé est un facteur essentiel du paramétrage. Ceci signifie qu'un contrôle de la cinétique d'acidification du milieu par la substance microbienne est nécessaire.

De façon générale, plus la cinétique d'acidification du milieu est rapide et plus l'indicateur changera d'état rapidement (à une température déterminée). Il convient donc de contrôler la cinétique d'acidification du milieu au sein de l'indicateur. Pour contrôler la cinétique d'acidification, il convient de contrôler principalement les éléments suivants :
(1) le choix de la substance microbienne par rapport à sa capacité à croître à la température de conservation du produit dont elle viendra stimuler l'évolution ;
(2) le choix de la substance microbienne par rapport à sa capacité à produire de l'acide ;
(3) la quantité de substance microbienne introduite dans le milieu ;
(4) la quantité de nutriments nécessaire au développement de la substance microbienne ;
(5) la quantité de substrat précurseur nécessaire à la production d'acide par la substance microbienne ;
(6) l'ensemble des paramètres pouvant affecter le développement de la substance microbienne ;
(7) le pH de départ du milieu ;
(1) La substance microbienne doit être choisie en fonction de sa cinétique de croissance et de son activité métabolique acidifiante selon la température de conservation du produit. Selon l'invention des souches psychrophiles sont choisies pour suivre la conservation de produits devant être gardés entre 0°C et +4°C, à savoir les souches CRYO-CB002, CRYO-CB001 tel qu'illustré par la figure 1.

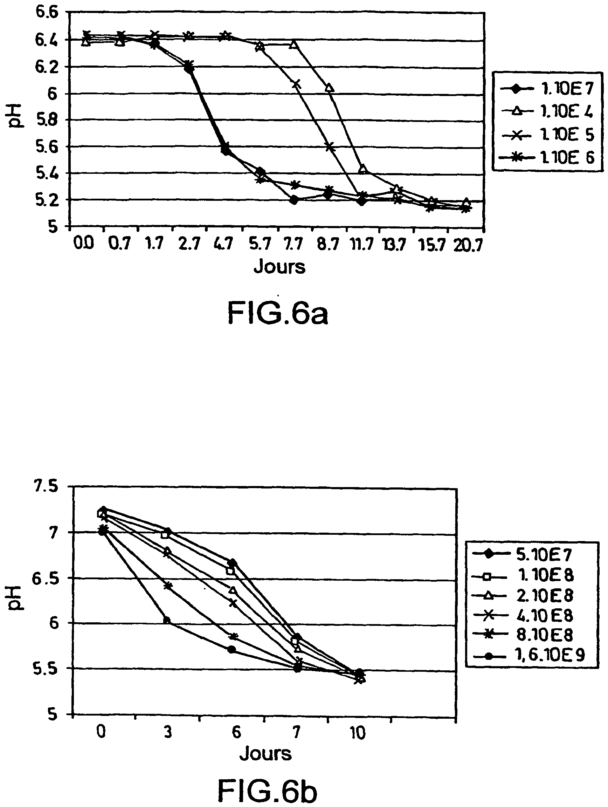

Plus la souche bactérienne choisie est psychrophile, plus l'indicateur va changer d'état rapidement à basse température (0°C, +4°C ou +8°C). De même, plus la souche choisie pousse lentement, moins sa production d'acide est rapide et moins l'indicateur va changer d'état rapidement à une température donnée.
(2) La quantité de substance microbienne introduite dans l'indicateur (inoculum) influe sur la cinétique d'acidification du milieu et donc son paramétrage. Plus la quantité de substance microbienne introduite dans l'indicateur est faible, et plus il faudra de temps pour que le milieu s'acidifie. A l'inverse, plus la quantité de substance microbienne introduite dans le milieu est importante et plus le milieu s'acidifie rapidement (pour une formulation de milieu donnée). Pour la souche CRYO-CB002, l'acidification est plus rapide à +4°C pour un inoculum à 1,6.10^{E}9 CFU/ml que pour un inoculum de 5.10^{E}7 CFU par ml de milieu, ainsi que présenté dans la Figure 6-b (l'indicateur, dans cet exemple, comprend 2,5% de gélatine bovine avec un pH de départ de 7,2). Avec cette même souche, dans des conditions de DLC 1/3 à +4°C et 2/3 à +8°C, l'acidification est plus rapide avec 1.10^{E}7 CFU/ml qu'avec 1.10^{E}4 CFU par ml de milieu, ainsi que présenté sur la. Figure 6-a (l'indicateur, dans cet exemple, comprend 2% de gélatine 200 de volaille avec un pH de départ de 6,4).

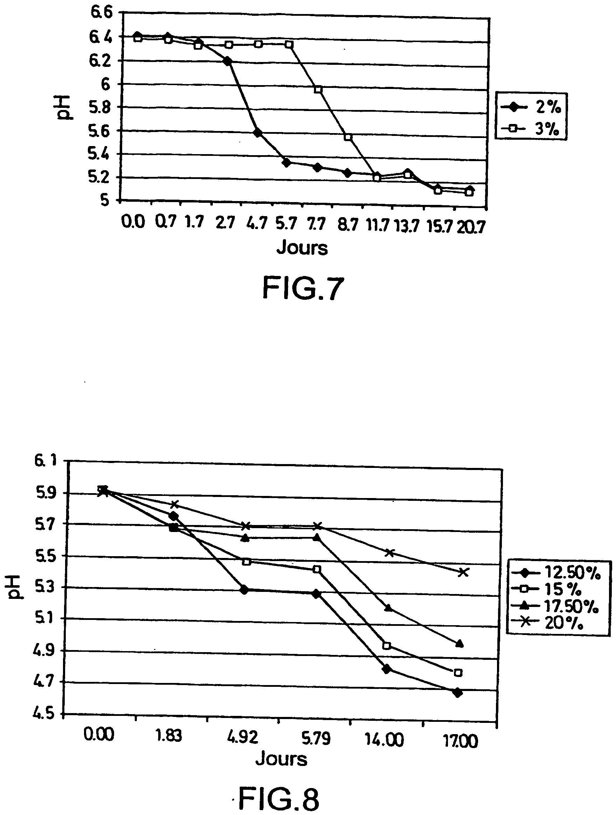

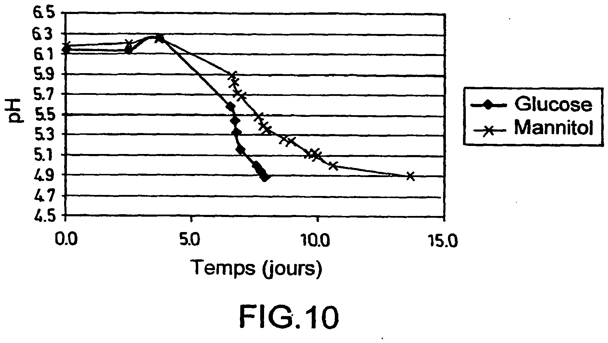

Ainsi pour la souche CRYO-CB002 et pour une formulation de milieu donnée, un inoculum de 10^{E}5 CFU/ml permet un virage de l'indicateur coloré et une précipitation de la caséine en 11 jours à +8°C alors qu'un inoculum de 10^{E}7 CFU/ml permet un virage et une précipitation en 7 jours dans les mêmes conditions.
(3) La quantité de nutriments permet de contrôler la vitesse de développement de la substance microbienne. Ainsi l'indicateur peut être paramétré en faisant varier la composition du milieu. Par exemple, la vitesse de croissance d'une souche bactérienne (et donc la vitesse d'acidification du milieu) dépend des nutriments présents et de leur concentration. Plus le milieu est favorable au développement microbien et plus l'acidification du milieu sera rapide. Ainsi, l'utilisation de Mannitol entraîne une acidification du milieu, par la souche CRYO-CB002, plus lente que dans le cas de l'utilisation de glucose ainsi que présenté dans la Figure 10 (l'indicateur comprend, dans cet exemple, un inoculum de 1.10^{E}4 CFU/ml de la souche CRYO-CB002 et 2% de glucose ou 2% de mannitol).
(4) La quantité de substrat nécessaire à la production d'acide par la substance microbienne permet de contrôler la vitesse d'acidification du milieu. En effet, si le substrat précurseur de l'acide fabriqué (par exemple un sucre fermentescible et par exemple le glucose pour une souche de bactérie lactique) est une condition limitante, il faudra plus de temps pour que la substance microbienne produise suffisamment d'acide pour faire chuter le pH en dessous d'un seuil déterminé.
(5) D'autres paramètres sont susceptibles d'influer sur le développement de la substance microbienne. Par exemple la qualité et la quantité de texturant utilisé dans l'indicateur. Par exemple, pour un gélifiant, plus la force du gel formé sera importante (à cause des propriétés du gélifiant ou à cause de sa concentration) et moins la souche pourra se développer rapidement ce qui ralentira la cinétique d'acidification du milieu. Par exemple, pour une formulation de milieu donnée et pour une souche donnée, l'acidification du milieu est plus lente avec 3% qu'avec 2% de gélatine de volaille (force de bloom 200), ainsi que présenté dans la Figure 7 (l'indicateur, dans cet exemple comprend un inoculum de 1.10E6 CFU/ml de la souche CRYO-CB002 avec de la gélatine 200 Volaille à 2 et 3% avec un pH de départ de 6,4 et pour des conditions de conservation de 1/3 du temps à +4°C et 2/3 à +8°C).

L'a" est également un paramètre important pour la vitesse de développement de la substance microbienne. L'a_{w} est l'activité de l'eau ; elle exprime la disponibilité de l'eau dans un milieu. Il est important de distinguer l'eau libre, chimiquement non liée (totalement disponible pour participer aux réactions) et l'eau liée (non disponible notamment pour les micro-organismes). L'a_{w} est inversement proportionnelle à la pression osmotique d'un composé. Ainsi, elle est affectée par la présence plus ou moins importante d'humectants (NaCl (sels), glycérol, sorbitol...) dissous dans l'eau. La disponibilité de l'eau présente dans un milieu ou dans une substance intervient dans la croissance bactérienne. L'abaissement de l'a_{w} ralentit l'activité enzymatique ; il augmente la durée de la phase de latence des microorganismes et diminue leur vitesse de croissance. Ainsi modifier la concentration en glycérol ou en tout autre humectant pour faire varier la valeur de l'a_{w}, comme illustré dans le tableau suivant, est intéressant et permet de modifier et maîtriser les temps de croissance des bactéries.

| A_{w} | Pourcentage de glycérol dans le milieu (en g) | Pourcentage de NaCl dans le milieu (en g) |
|---|---|---|
| 0.995 | 0 | 0 |
| 0.990 | 3 | 1 |
| 0.985 | 6 | 2.5 |
| 0.980 | 10 | 3.5 |
| 0.975 | 13.5 | 4.5 |
| 0.970 | 15 | 5 |
| 0.960 | 20 | 6.5 |

| | | |
|---|---|---|
| a_{w} correspondant au pourcentage de NaCl ou de Glycérol présent dans la milieu | | |

De façon générale, abaisser l'a_{w} du milieu, en deçà de la valeur d'a_{w} optimale d'une souche, ralentit la cinétique d'acidification du milieu. Par exemple, plus la concentration en glycérol ou en NaCl est importante dans le milieu et plus le changement d'état de l'indicateur sera ralenti (pour une formulation d'indicateur donnée). Par exemple, à +8°C, avec la souche CRYO-CB002, l'acidification est plus lente si le milieu contient 20% de glycérol que s'il en contient 12,5%, ainsi que présenté dans la Figure 8.
(6) Enfin, le pH de départ du milieu est important pour paramétrer l'indicateur.

D'une part, le pH du milieu doit être compatible avec le développement et l'activité métabolique de la substance microbienne. Ainsi pour la souche CRYO-CB002, le pH doit être compris entre 3,6 et 9,2. Le pH du milieu influe sur la vitesse de développement de la substance microbienne (par exemple des bactéries lactiques) et donc sur son activité acidifiante. Les meilleurs développement microbiens, et donc les cinétiques d'acidification les plus rapides, sont obtenus en se plaçant à des pH proches du pH optimal de croissance de la souche choisie (par exemple pH 7,1 pour la souche CRYO-CB002).

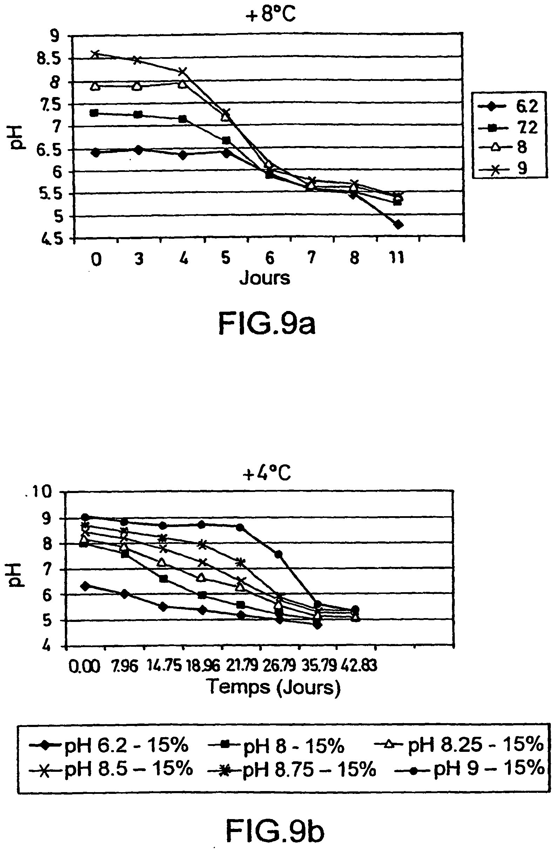

D'autre part, le changement d'état de l'indicateur provenant du passage du pH en dessous d'une valeur seuil, plus le pH de départ du milieu est éloigné du pH seuil et plus il faudra de temps pour que le pH du milieu descende (grâce à l'activité acidifiante de la substance microbienne) jusqu'à cette valeur seuil. Autrement dit, plus le pH de l'indicateurs sera élevé (et supérieur au pH optimal de croissance de la souche) et plus le changement d'état de l'indicateur arrivera tardivement (pour une température donnée). Par exemple, l'acidification du milieu par la souche CRYO-CB002 arrive plus tardivement si le pH de départ est de 9 que s'il est de 6,2 ainsi que présenté dans la Figure 9-a à +8°C (l'indicateur comprend, dans cet exemple, 2,5% de gélatine 200 de volaille et un inoculum 1.10^{E}5 CFQ/ml de la souche CRYO-CB002) et la Figure 9-b à +4°C (l'indicateur comprend, dans cet exemple, un inoculum 1.10^{E}6 CFU/ml de la souche CRYO-CB002 et 15% de glycérol) .

Enfin, si le pH de départ du milieu est tamponné à un pH donné (avec un tampon Phosphate ou Tris par exemple), le pH de l'indicateur descendra plus lentement ce qui retardera le moment auquel le ou les révélateurs changeront d'état. Par exemple à +8°C, pour un milieu 1% agar à pH 6,2 ; contenant 2% de mannitol ; 15% de glycérol ; 5% de caséinate de Sodium ; 3% d'Indicateur mixte; inoculé à 1.10^{E}4 CFU/ml avec la souche CRYO-CB002, le temps de précipitation est de 12 jours lorsque le pH de départ est ajusté avec de l'HCl et du NaOH, et le temps de précipitation est de 17 jours si un tampon phosphaté 0,1M est utilisé.

Pour le paramétrage, nous avons évoqué les principaux éléments, entrant dans la fabrication de l'indicateur et influant sur la vitesse de croissance de la substance microbienne ainsi que sur la vitesse d'acidification du milieu par ladite substance microbienne.

La vitesse d'acidification du milieu va déterminer le tempes nécessaire, à une température donnée, pour que le pH du milieu atteigne la valeur seuil à laquelle le ou les révélateurs vont changer d'état (par exemple une précipitation de la caséine ou un virage colorimétrique d'un indicateur coloré).

Afin d'illustrer les données sur le paramétrage de l'indicateur et de donner les informations nécessaires à la bonne mise au point de celui-ci, voici une série d'exemples de formulation de l'indicateur en fonction des dates limites de consommation, ou d'utilisation optimale, relatives à différentes températures de conservation.

Pour ces exemple, les inventeurs se sont attachés à valider des Dates Limites de Consommation de produits périssables telles qu'elles ont été préalablement définies par l'industriel dans les conditions de la norme AFNOR NF V 01-003 en paramétrant l'indicateur sur ladite Date

| mc (temps d'opacification de l'indicateur | Condition Température | Souche | Inicolum | pH Départ | Milieu |
|---|---|---|---|---|---|
| 7 jours | 1/3 +4°C ; 2/3 +8°C | CRYO-CB002 | 1.10^{E}4 | 6,2 | Glucosa 2% Agar 1% Caséinate Na+ 5% |
| 10 Jours | 1/3 +4°C ; 2/3 +8°C | CRYO-CB002 | 1.10^{E}4 | 6,2 | Glucose 2% Glycérol 15% Agar 1% Caséinata Na+ 5% |
| 20 jours | 1/3 +4°C 2/3 +8°C | CRYO-CB002 | 1.10^{E}4 | 6,2 | Mannitol 2% Glycérol 20% Agar 1% Tampon Phosphata 0,1M Caséinate Na+ 5% |
| 11 jours | 2/3 +4°C ; 1/3 +8°C | CRYO-CB002 | 1.10^{E}4 | 6,5 | Glucose 2% Scléroglucane 1% Caséinate Na+ 10% |
| 13 jours | +4°C | CRYO-CB002 | 1.10^{E}4 | 6,5 | Glucose 2% Xanthane 0,25% Caséinate Na+ 10% |
| 10 jours | 2/3 +4°C ; 1/3 +8°C | CRYO-CB002 | 1.10^{E}7 | | Glucose 2% Gélatine 310 Porc 1,6% Caséinate Na+ 10% |
| 9 jours | +8°C | CRYO-CB002 | 1.10^{E}5 | 6,2 | Glucose 2% Gélatine 310 Porc 1,2% Caséinate Na+ 10% |
| 21 jours | +4°C | CRYO-CB002 | 1.10^{E}5 | 6,2 | Glucose 2% Gélatine 310 Porc 1,2% Caséinate Na+ 10% |
| 16 jours | +8°C | CRYO-CB002 | 1.10^{K}5 | 6,4 | Glucose 2% Gélatine 310 Porc 5% Caséinate Na+ 10% |

L'invention n'est pas imitée aux modes de réalisation décrits et l'homme du métier reconnaîtra l'existence de diverses variantes de réalisation comme par exemple l'utilisation au sein de l'indicateur d'une sélection de souches qui résistent aux basses températures pour suivre l'évolution d'un produit qui pourrait subir une étape de congélation, ou encore d'une sélection de souches spécifiques à des températures plus élevées que celle présentées dans cette description, ou encore l'utilisation d'un mélange de souches sélectionnées.

Selon un autre de ses aspects, l'invention concerne un procédé pour contrôler la date limite réelle de consommation d'un produit périssable, cette date limite étant atteinte lorsque le produit a été conservé pendant une certaine durée dans des conditions de température préconisées, procédé selon lequel on associe au produit un composant indicateur comprenant :
- un microorganisme présentant des caractéristiques de croissance déterminées dans les conditions de température préconisées pour ledit produit, ces caractéristiques étant évolutives en fonction du temps et de la température ;
- un milieu de culture pour le microorganisme, et
- un agent révélateur dépendant d'un seuil de croissance du microorganisme,
de sorte que l'indicateur fournit une indication de changement d'état à la date limite préconisée lorsque le produit a été conservé dans les conditions de température préconisées, ou avant cette date ou après cette date, lorsque le produit a été conservé dans des conditions de température moins bonnes ou meilleures, respectivement.

Dans une réalisation, le microorganisme est un microorganisme produisant un acide, et l'agent révélateur est dépendant du pH du milieu.

Dans une réalisation, l'agent révélateur est la caséine, qui forme un précipité lorsque le pH du milieu descend au-dessous d'une valeur prédéterminée.

L'agent révélateur peut être un indicateur coloré dépendant du pH.

Selon l'invention, le microorganisme est une population de bactéries lactiques du genre carnobacterium choisie parmi la souche CRYO-CB001 et la souche CRY-CB002.

Dans ce cas, le microorganisme peut être une population de bactéries lactiques pouvant pousser à des températures inférieures à +8°C, de préférence inférieures à +6°C, plus préférentiellement à une température de +4°C ou moins. Le microorganisme peut être une population de bactéries lactiques pouvant pousser à des températures comprises entre - +4°C et +50°C.

Dans une réalisation, le microorganisme peut être congelé et décongelé.

Dans une réalisation, le milieu de culture comprend des nutriments nécessaires à la survie et au développement des microorganismes.

Dans une réalisation, le milieu de culture comprend en outre des précurseurs nécessaires à une fonction acidifiante.

Dans une réalisation, le milieu de culture comprend en outre un agent texturant.

Dans une réalisation, le composant indicateur est sous forme d'une étiquette, éventuellement adhésive.

Dans ce cas, le composant indicateur peut être inclus dans un film, dont une face au moins comprend une zone permettant d'observer le signal produit par l'agent révélateur, comprenant une quantité prédéterminée de microorganisme acidifiant dans un milieu de culture approprié, un agent révélateur. L'étiquette peut aussi comprendre plusieurs compartiments internes, par exemple des capsules, permettant de séparer un ou plusieurs constituants du composant des autres pendant une durée déterminée, les parois desdits compartiments pouvant être rompues par tout moyen adapté, par exemple par pression sur l'étiquette.

Dans une réalisation, le produit périssable est un produit alimentaire ou pharmaceutique. Ce produit périssable est par exemple un produit alimentaire destiné à être conservé à une température de +4°C environ, et dans lequel le composant indicateur comprend un microorganisme capable de croissance à une température inférieure ou égale à +4°C.

L'invention concerne aussi un composant indicateur biologique caractérisé en ce qu'il comprend, dans un conditionnement, une population de microorganismes acidifiants présentant des caractéristiques de croissance évolutives en fonction du temps et de la température, un milieu de culture pour le microorganisme, un agent révélateur dépendant du pH du milieu, et un agent texturant, une zone de surface au moins du conditionnement permettant l'observation du signal produit par l'agent révélateur.

Dans une réalisation, les microorganismes sont présents dans un compartiment isolé, par exemple des capsules.

Dans une réalisation, les constituants sont choisis pour permettre la production d'un signal par l'agent révélateur après conservation du composant pendant une durée pré-définie à une température pré-définie.

Dans une réalisation, l'agent révélateur est la caséine, qui forme un précipité lorsque le pH du milieu descend au-dessous d'une valeur prédéterminée.

Dans une réalisation, l'agent révélateur est un indicateur coloré dépendant du pH.

Dans une réalisation, le microorganisme est une population de bactéries lactiques du genre carnobacterium choisie parmi la souche CRYO-CB001 et la souche CRYO-CB002 .

Dans une réalisation, le microorganisme est une population de bactéries lactiques pouvant pousser à des températures inférieures à +8°C, de préférence inférieures à +6°C, plus préférentiellement à une température de 4°C ou moins.

Dans une réalisation, le microorganisme est une population de bactéries lactiques pouvant pousser à des températures comprises entre -4°C et +50°C.

Dans une réalisation, le microorganisme peut être congelé et décongelé.

Dans une réalisation, le milieu de culture comprend des nutriments nécessaires à la survie et au développement des microorganismes, ainsi qu'à la fonction acidifiante.

L'invention concerne aussi un procédé de préparation d'un composant indicateur biologique défini ci-dessus comprenant l'obtention d'une population de microorganismes acidifiants présentant des caractéristiques de croissance évolutives en fonction du temps et de.la température et, le conditionnement de ces microorganismes dans un milieu de culture approprié en présence d'un agent révélateur dépendant du pH du milieu et le cas échéant d'un agent texturant, dans un emballage dont une zone de surface au moins permet l'observation du signal produit par l'agent révélateur.

Dans une réalisation, certains constituants de l'indicateur sont placés dans des compartiments séparés, dont les parois peuvent être rompues.

L'invention concerne aussi l'ensemble formé par un produit périssable et un composant indicateur tel que défini ci-dessus qui est associé, notamment par opposition, à ce produit.

Dans une réalisation, le composant indicateur est apposé par des moyens adhésifs.

Dans une réalisation, le composant indicateur comporte plusieurs compartiments et est activé après, ou lors de l'apposition, par rupture des parois des compartiments.

Dans une réalisation, le produit périssable est un produit alimentaire ou pharmaceutique.

## Revendications

1. Procédé pour déterminer si un produit est ou non en état d'être utilisé ou consommé, ce produit étant destiné à être conservé au dessous d'une température limite dans des conditions de température et pendant une durée limitant sa dégradation, procédé dans lequel on associe au produit un indicateur tel qu'il fournit un signal indiquant la possibilité d'utilisation ou de consommation du produit, puis, après l'expiration d'une durée de conservation, un signal indiquant un changement d'état de possibilité d'utilisation ou de consommation du produit, cette durée évoluant en fonction des conditions de conservation du produit,
l'indicateur étant tel qu'il fournit une information permettant que le produit soit utilisable ou consommable pendant une durée plus importante si le produit a été conservé dans des conditions meilleures que des conditions standards ou préconisées, ou pendant une durée moins importante si le produit a été conservé dans des conditions moins bonnes que des conditions standards ou préconisées,
l'indicateur contenant au moins une substance microbienne ayant une activité acidifiante faisant varier le pH du milieu dans lequel elle se trouve;
ladite substance microbienne contenant au moins une souche de bactéries choisie parmi :
- la souche CRYO-CB001 de l'espèce *Carnobacterium piscicola* enregistrée auprès de la CNCM (Collection Nationale de Culture de Microorganismes, Paris, France) sous le N° CNCM I-3297, le 16 septembre 2004 ; et
- la souche CRYO-CB002 de l'espèce *Carnobacterium piscicola* enregistrée auprès de la CNCM (Collection Nationale de Culture de Microorganismes, Paris, France) sous le N° CNCM 1-3298, le 16 septembre 2004 ;
et dans lequel on produit, à l'aide d'un révélateur, le signal indiquant le changement d'état quand le pH du milieu dans lequel se trouve la substance microbienne descend au-dessous d'une valeur prédéterminée.

2. Procédé selon la revendication 1 dans lequel le révélateur forme un précipité lorsque le pH descend au-dessous de la valeur prédéterminée.

3. Procédé selon la revendication 2 dans lequel le révélateur comporte de la caséine.

4. Procédé selon l'une des revendications 1 ou 2 dans lequel le révélateur comporte au moins un indicateur coloré.

5. Procédé selon la revendication 4 dans lequel l'indicateur coloré est choisi dans le groupe suivant : Alizarine, Alizarine Sodium Sulfonate, Alzarine Red , Benzopurpurin, Benzoyl Auramine G, Benzoylethylauramine, Bleu De Résorcine, Bleu De Bromochlorophénol, Bromocresol Green, Bromophenol Blue, Carmine Acid, 2,4-Dinitrophenol, 4- (4-Dimethylamino-1-Naphtylazo) -3-Methoxybenzenesulfonic Acid, α- dinitrophénol, β-dinitrophénol, γ-dinitrophénol, Disodium4,4- Bis (O-Tolytriazeno)-2, 2'-Stilbenedisulfonate, Disodium4,4- Bis (P-Dimethytaminophenylazo)-2, 2'-Stilbenedisulfonate, 4-(P-Ethoxyphenylazo)-M-Phenylene-Diamine Monohydrochloride, Ethyl Red, Ethyl Orange, Hexamethoxy Red, Hydroquinolsulphonaphtalein, Lacmoid, Iodeosin, Lasmoid, N, Ndimethyl-P- (M-Tolylazo) Aniline, 4'-Methoxy-2. 4-Diaminoazobenzene, Methyl Red, Methyl Red- Alphazurin, α-Naphtylamine, α-Naphtylaminoazo-Benzène, Naphtyl Red, Oxime Blue, 4'-Oxy-3'-Methyl-2. 4-Diaminoazobenzene, 4'-Oxy- 2.4diaminoazobenzène, 4-Phenylazo-1-Naphtylamine, Parafuchsine- Hexa-Acetic Acid, Paranitrophénol, P-Ethoxychrysoidine, P-Sulfo- O-Methoxybenzeneazodimethyl-aNaphtylamine, Rouge Congo, Red Violet, Resazurin, Rouge Naphtyl, Tetrabromocresol, Tétraiodophenolsulfophthalein, acide aurine, dithiozone, bleu de chrome, bromocrésol pourpre, carmin indigo, cacotheline, calcéine, eosine extra, eriochromocyanine, jaune thiazol, noir d'eriochrome T, α-nitro β-naphtol, orange **III,** rouge methyl, rhodamine B, rhodizonate de potassium, thymolsulffonephtalein, rhodium chlorure, trihydroxy2, 6, 7 phenyl-9-isoxanthen, thorin, xylene orange tetranatrium salz, anthocyanes, phénolphtaléine, le benzopurpurin 4B, benzopurpurin B, alpha naphtyl red hydrochloride, litmus, Rouge de méthyle, Indicateur Mixte, Lacmoïd.

6. Procédé selon l'une des revendications 1 à 5 dans lequel, pour adapter Indicateur au produit, on choisit au moins un des paramètres compris dans le groupe suivant : la nature de la substance microbienne, la quantité de cette substance microbienne, la nature des nutriments, la quantité des nutriments, la nature des éléments nécessaires à la production d'acide par la substance microbienne et la quantité de ces éléments, la nature d'un texturant du milieu dans lequel se trouve la substance microbienne et la quantité de ce texturant, le pH de départ du milieu, les paramètres influant sur l'aw du milieu, la nature du révélateur utilisé pour mesurer la baisse du pH et la quantité de ce révélateur, les paramètres déterminant le pH auquel ce révélateur change d'état.

7. Procédé selon la revendication 6, dans lequel les paramètres sont choisis pour que le signal indiquant le changement d'état ait lieu au bout d'une durée prédéterminée lorsque le produit est conservé dans des conditions standards ou préconisées.

8. Procédé selon la revendication 6, dans lequel les paramètres sont choisis pour que le signal indiquant le changement d'état apparaisse lorsque le produit n'est plus consommable.

9. Procédé selon l'une des revendications 7 ou 8 dans lequel, dans des conditions standards ou préconisées, on choisit les paramètres de l'indicateur pour que le signal indiquant le changement d'état ait lieu après une durée déterminée, cette durée étant augmentée en modifiant un de ces paramètres en l'éloignant des valeurs optimales pour l'acidification par la substance microbienne.

10. Procédé selon l'une des revendications 6 à 9 dans lequel les nutriments incluent une source de carbone et une source d'azote, et, de préférence, des sels minéraux et/ou des vitamines et/ou des oligoéléments.

11. Procédé selon la revendication 10 dans lequel la source de carbone est choisie parmi les sucres du groupe suivant : Glycérol, Erythritol, D-Arabinose, L-Arabinose, Ribose, D-Xylose, L-Xylose, Adonitol, Methyl- xyloside, Galactose, D-Glucose, D-Fructose, D-Mannose, L-Sorbose, Rhamnose, Dulcitol, Inositol, Mannitol, Sorbitol, α Methyl-D-mannoside, (α Methyl-D-glucoside, N Acetyl glucosamine, Amygdaline, Arbutine, Esculine, Salicine, Cellobiose, Maltose, Lactose, Melibiose, Saccharose, Trehalose, Inuline, Melezitose, D-Raffinose, Amidon, Glycogène, Xylitol, βGentiobiose, D-Turanose, D-Lyxose, D-Tagatose, D-Fucose, L- Fucose, D-Arabitol, L-Arabitol, Gluconate, 2 ceto-gluconate, 5 ceto-gluconate.

12. Procédé selon l'une des revendications 6 à 11 dans lequel un texturant est choisi parmi ceux du groupe suivant : agar, agarose, gélatine, xanthane, scléroglucane, Gum Guar.

13. Procédé selon l'une quelconque des revendications précédentes dans lequel l'indicateur est inactivé quand il n'est pas associé au produit et activé lorsqu'il est associé au produit

14. Procédé selon la revendication 13 dans lequel l'inactivation est réalisable à l'aide de moyens choisis dans le groupe suivant : congélation, microencapsulation de la substance microbienne et/ou des nutriments, compartimentation.

15. Procédé selon la l'une des revendications 13 ou 14 dans lequel l'activation de l'indicateur est réalisée par une action physique choisie dans le groupe suivant : variation de pression, variation de température, variation de longueur d'onde d'exposition à un rayonnement.

16. Procédé selon l'une quelconque des revendications 1 à 15 dans lequel la substance microbienne est congelée ou décongelée.

17. Application du procédé selon l'une quelconque des revendications précédentes à des produits de type alimentaire ou biologique ou pharmaceutique.

18. Application du procédé selon l'une quelconque des revendications 1 à 16 à des produits alimentaires dans lequel l'indicateur est paramétré pour que l'apparition du signal indiquant un changement d'état soit d'autant plus retardée que la température de conservation du produit est proche de la température optimale de conservation.

19. Application du procédé selon l'une quelconque des revendications 1 à 16 à un produit alimentaire destiné à être conservé à des températures comprises entre 0°C et +4°C environ, et dans lequel l'indicateur comprend une substance microbienne ayant une activité acidifiante à des températures inférieures ou égales à +4°C environ.

20. Dispositif pour déterminer si un produit est ou non en état d'être utilisé ou consommé, ce produit étant destiné à être conservé au dessous d'une température limite dans des conditions de température et pendant une durée limitant sa dégradation, ce dispositif comprenant au moins un indicateur destiné à être associé au produit, cet indicateur étant tel qu'il fournit un signal indiquant la possibilité d'utilisation ou de consommation du produit, puis, après l'expiration d'une durée de conservation, un signal indiquant un changement d'état de possibilité d'utilisation ou de consommation du produit, cette durée évoluant en fonction des conditions de conservation du produit,
l'indicateur étant tel qu'il fournit une information permettant que le produit soit utilisable ou consommable pendant une durée plus importante si le produit a été conservé dans des conditions meilleures que des conditions standards ou préconisées, ou pendant une durée moins importante si le produit a été conservé dans des conditions moins bonnes que des conditions standards ou préconisées,
l'indicateur contenant au moins une substance microbienne ayant une activité acidifiante faisant varier le pH du milieu dans lequel elle se trouve;
ladite substance microbienne contenant au moins une souche de bactéries choisie parmi :
- la souche CRYO-CB001 de l'espèce *Carnobacterium piscicola* enregistrée auprès de la CNCM (Collection Nationale de Culture de Microorganismes, Paris, France) sous le N° CNCM 1-3297, le 16 septembre 2004 ; et
- la souche CRYO-CB002 de l'espèce *Carnobacterium piscicola* enregistrée auprès de la CNCM (Collection Nationale de Culture de Microorganisrnes, Paris, France) sous le N° CNCM 1-3298, le 16 septembre 2004 ;
ledit indicateur contenant en outre, un révélateur et des moyens pour fournir un signal indiquant le changement d'état quand le pH du milieu dans lequel se trouve la substance microbienne descend au-dessous d'une valeur prédéterminée.

21. Dispositif selon la revendication 20 dans lequel le révélateur est tel qu'il forme un précipité lorsque le pH descend au-dessous de la valeur prédéterminée.

22. Dispositif selon la revendication 21 dans lequel le révélateur comporte de la caséine.

23. Dispositif selon l'une des revendications 20 ou 21 dans lequel le révélateur comporte au moins un indicateur coloré.

24. Dispositif selon la revendication 23 dans lequel l'indicateur coloré est compris dans le groupe suivant : Alizarine, Alizarine Sodium Sulfonate, Alzarine Red , Benzopurpurin, Benzoyl Auramine G, Benzoylethylauramine, Bleu De Résorcine, Bleu De Bromochlorophénol, Bromocresol Green, Bromophenol Blue, Carmine Acid, 2,4-Dinitrophenol, 4- (4- Dimethylamino-1-Naphtylazop3-Methoxybenzenesulfonic Acid, α-dinitrophénol, β-dinitrophénol, γ-dinitrophénol, Disodium4, 4- Bis (O-Tolytriazeno)-2, 2'-Stilbenedisulfonate, Disodium4,4- Bis (P-Dimethylaminophenylazo)-2, 2'-Stilbenedisulfonate, 4-(P- Ethoxyphenylazo) -M-Phenylene-Diamine Monohydrochloride, Ethyl Rcd, Ethyl Orange, Hexamethoxy Rcd, Hydroquinolsulphonaphtalein, Lacmoid, Iodeosin, Lasmoid, N, Ndimethyl-P- (M-Tolylazo) Aniline, 4'-Methoxy-2. 4-Diaminoazobenzene, Methyl Red, Methyl Red- Alphazurin, α-Naphtylamine, α-Naphtylaminoazo-Benzène, Naphtyl Red, Oxime Blue, 4'-Oxy-3'-Methyl-2. 4-Diaminoazobenzene, 4'-Oxy- 2.4diaminoazobenzène, 4-Phenylazo-l-Naphtylamine, Parafuchsine- Hexa-Acetic Acid, Paranitrophénol, P-Ethoxychrysoidine, P-Sulfo- O-Methoxybenzeneazodimethyl-αNaphtylamine, Rouge Congo, Red Violet, Resazurin, Rouge Naphtyl, Tetrabromocresol, Tétraiodophenolsulfophthalein, acide aurine, dithiozone, bleu de chrome, bromocrésol pourpre, carmin indigo, cacotheline, calcéine, eosine extra, eriochromocyanine, jaune thiazol, noir d'eriochrome T, α-nitro β-naphtol, orange III, rouge methyl, rhodamine B, rhodizonate de potassium, thymolsulffonephtalein, rhodium chlorure, trihydroxy2, 6, 7 phenyl-9-isoxanthen, thorin, xylene orange tetranatrium salz, anthocyanes, phénolphtaléine, le benzopurpurin 4B, benzopurpurin B, alpha naphtyl red hydrochloride, litmus, Rouge de méthyle, Indicateur Mixte, Lacmoïd.

25. Dispositif selon l'une quelconque des revendications 20 à 24 dans lequel au moins un des paramètres de l'indicateur est tel que l'indicateur soit adapté au produit, les paramètres étant compris dans le groupe suivant : la nature de la substance microbienne, la quantité de cette substance microbienne, la nature des nutriments, la quantité de ces nutriments, la nature des éléments nécessaire à la production d'acide par la substance ,. microbienne et la quantité de ces éléments, la nature d'un texturant du milieu dans lequel se trouve la substance microbienne et la quantité de ce texturant, le pH de départ du milieu, les paramètres influant sur Ilaw du milieu, la nature du révélateur utilisé pour mesurer la baisse du pH, et la quantité de ce révélateur, les paramètres déterminant le pH auquel ce révélateur change d'état.

26. Dispositif selon la revendication 25 dans lequel l'indicateur est tel que les paramètres sont tels que le signal indiquant le changement d'état ait lieu au bout d'une durée prédéterminée lorsque le produit est conservé dans des conditions standards ou préconisées.

27. Dispositif selon la revendication 25 comprenant un indicateur dans lequel les paramètres sont tels que le signal indiquant le changement d'état apparaît lorsque le produit n'est plus consommable ou utilisable.

28. Dispositif selon l'une quelconque des revendications 25 à 27 dans lequel les nutriments incluent une source de carbone et une source d'azote.

29. Dispositif selon l'une quelconque des revendications 20 à 28, **caractérisé en ce qu'**il contient un milieu de culture contenant une source de carbone et une source d'azote.

30. Dispositif selon l'une des revendications 28 ou 29 dans lequel la source de carbone est comprise parmi les sucres du groupe suivant : Glycérol, Erythritol, D-Arabinose, L-Arabinose, Ribose, D-Xylose, L-Xylose, Adonitol, Methyl- xyloside, Galactose, D-Glucose, D-Fructose, D-Mannose, L- Sorbose, Rhamnose, Dulcitol, Inositol, Mannitol, Sorbitol, α Methyl-D-mannoside, (α Methyl-D-glucoside, N Acetyl glucosamine, Amygdaline, Arbutine, Esculine, Salicine, Cellobiose, Maltose, Lactose, Melibiose, Saccharose, Trehalose, Inuline, Melezitose, D-Raffinose, Amidon, Glycogène, Xylitol, ßGentiobiose, D-Turanose, D-Lyxose, D-Tagatose, D-Fucose, L- Fucose, D-Arabitol, L-Arabitol, Gluconate, 2 ceto-gluconate, 5 ceto-gluconate.

31. Dispositif selon l'une quelconque des revendications 25 à 30 dans lequel un texturant est compris parmi ceux du groupe suivant : agar, agarose, gélatine, xanthane, scléroglucane, Gum Guar.

32. Dispositif selon l'une quelconque des revendications 1 à 31, **caractérisé en ce qu'**il comprend un texturant choisi dans le groupe suivant : agar, agarose, gélatine, xanthane, scléroglucane, Gum Guar.

33. Dispositif selon l'une quelconque des revendications 20 à 32 dans lequel l'indicateur est tel qu'il est inactivé quand il n'est pas associé au produit et activé lorsqu'il est associé au produit.

34. Dispositif selon la revendication 33 tel que l'inactivation est réalisable à l'aide de moyens compris dans le groupe suivant : congélation, microencapsulation de la substance microbienne et/ou des nutriments, compartimentation.

35. Dispositif selon l'une des revendications 33 ou 34 tel que l'activation de l'indicateur est réalisable par une action physique choisie dans le groupe suivant : variation de pression, variation de température, variation de la longueur d'onde d'exposition à un rayonnement.

36. Dispositif selon l'une des revendications 20 à 35 dans lequel la substance microbienne est congelable et décongelable.

37. Dispositif selon l'une des revendications 20 à 36 dans lequel l'indicateur se présente sous la forme d'une étiquette, de préférence autocollante.

38. Dispositif selon la revendication 37 dans lequel l'indicateur comprend au moins une face dont une zone permet d'observer le signal produit par le révélateur.

39. Dispositif selon l'une des revendications 37 ou 38 dans lequel l'étiquette comprend plusieurs compartiments internes, par exemple des capsules, permettant de séparer un ou plusieurs constituants du composant des autres pendant une durée déterminée, les parois desdits compartiments pouvant être rompues par tout moyen adapté, par exemple par pression sur l'étiquette.

## Claims

1. Method for determining whether or not a product is in a condition for use or consumption, this product being intended for preservation below a limit temperature under temperature conditions and for a period of time which limits its degradation, wherein an indicator is associated with the product such that it provides a signal indicating the possibility of use or consumption of the product and then, after expiry of a preservation period of time, a signal indicating a change in the condition of the possibility of use or consumption of the product, this period of time evolving as a function of the conditions of preservation of the product,
the indicator being such that it provides an information which enables the product to be used or consumed for a longer period of time if the product has been preserved under conditions better than the standard or recommended conditions, or for a shorter period of time if the product has been preserved under conditions poorer than the standard or recommended conditions,
the indicator containing at least one microbial substance having an acidifying activity which varies the pH of the medium containing it,
the said microbial substance containing at least one strain of bacteria chosen from:
- the strain CRYO-CB001 of the species *Carnobacterium piscicola* registered at the CNCM (Collection Nationale de Culture de Microorganismes, Paris, France) under no. CNCM 1-3297 on 16th September 2004; and
- the strain CRYO-CB002 of the species *Carnobacterium piscicola* registered at the CNCM (Collection Nationale de Culture de Microorganismes, Paris, France) under no. CNCM 1-3298 on 16th September 2004;
and wherein , with the aid of a developer, the signal indicating the change of state is produced when the pH of the medium containing the microbial substance falls below a predetermined value.

2. Method according to claim 1, wherein the developer forms a precipitate when the pH falls below the predetermined value.

3. Method according to claim 2, wherein the developer comprises casein.

4. Method according to one of claims 1 or 2, wherein the developer comprises at least one coloured indicator.

5. Method according to claim 4, wherein the coloured indicator is chosen from the following group:
alizarin, alizarin sodium sulfonate, alizarin red, benzopurpurin, benzoylauramin G, benzoylethylauramin, resorcinol blue, bromochlorophenol blue, bromocresol green, bromophenol blue, carmine acid, 2,4-dinitrophenol, 4-(4-dimethylamino-1-naphthylazo)-3-methoxybenzenesulfonic acid, α-dinitrophenol, β-dinitrophenol, γ-dinitrophenol, disodium 4,4-bis(o-tolyltriazeno)-2,2'-stilbenedisulfonate, disodium 4,4-bis(p-dimethylaminophenylazo)-2,2'-stilbenedisulfonate, 4-(p-ethoxyphenylazo)-m-phenylenediamine monohydrochloride, ethyl red, ethyl orange, hexamethoxy red, hydroquinol sulfonaphthalein, lacmoid, iodeosin, lasmoid, N,N-dimethyl-p-(m-tolylazo)aniline, 4'-methoxy-2,4-diaminoazobenzene, methyl red, methyl red alphazurin, α-naphthylamine, α-naphthylaminoazobenzene, naphthyl red, oxime blue, 4'-oxy-3'-methyl-2,4-diaminoazobenzene, 4'-oxy-2,4-diaminoazobenzene, 4-phenylazo-1-naphthylamine, parafuchsin-hexaacetic acid, paranitrophenol, p-ethoxychrysoidine, p-sulfo-o-methoxybenzeneazodimethyl-α-naphthylamine, Congo red, red violet, resazurin, naphthyl red, tetrabromocresol, tetraiodophenolsulfophthalein, aurin acid, dithiozone, chromium blue, bromocresol purple, indigo carmine, cacotheline, calcein, eosin extra, eriochromocyanine, thiazole yellow, eriochrome black T, α-nitro-β-naphthol, orange III, methyl red, rhodamine B, potassium rhodizonate, thymolsulfonephthalein, rhodium chloride, 2,6,7-trihydroxy-9-phenylisoxanthene, thorin, xylene orange tetrasodium salt, anthocyanes, phenolphthalein, benzopurpurin 4B, benzopurpurin B, alpha-naphthyl red hydrochloride, litmus, methyl red, mixed indicator, lacmoid.

6. Method according to one of claims 1 to 5, wherein, in order to adapt the indicator to the product, at least one of the parameters included in the following group is chosen: the nature of the microbial substance, the amount of this microbial substance, the nature of the nutrients, the amount of the nutrients, the nature of the elements necessary for production of acid by the microbial substance and the amount of these elements, the nature of a texturizing agent of the medium containing the microbial substance and the amount of this texturizing agent, the starting pH of the medium, parameters which influence the water activity of the medium, the nature of the developer used to measure the drop in pH and the amount of this developer, parameters which determine the pH at which this developer changes condition.

7. Method according to claim 6, wherein the parameters are chosen such that the signal indicating the change of state takes place after a predetermined period of time when the product is preserved under standard or recommended conditions.

8. Method according to claim 6, wherein the parameters are chosen such that the signal indicating the change of state appears when the product can no longer be consumed.

9. Method according to claim 7 or 8, wherein, under standard or recommended conditions, the indicator parameters are chosen such that the signal indicating the change of state takes place after a given period of time, this period of time being increased by modifying one of these parameters, moving away from optimum values for acidification by the microbial substance.

10. Method according to one of claims 6 to 9, wherein the nutrients include a source of carbon and a source of nitrogen, and preferably mineral salts and/or vitamins and/or oligoelements.

11. Method according to claim 10, wherein the source of carbon is chosen from the sugars of the following group: glycerol, erythritol, D-arabinose, L-arabinose, ribose, D-xylose, L-xylose, adonitol, methyl xyloside, galactose, D-glucose, D-fructose, D-mannose, L-sorbose, rhamnose, dulcitol, inositol, mannitol,
sorbitol, α-methyl D-mannoside, α-methyl D-glucoside, N-acetylglucosamine, amygdalin, arbutin, esculin, salicin, cellobiose, maltose, lactose, melibiose, saccharose, trehalose, inulin, melezitose, D-raffinose, starch, glycogen, xylitol, β-gentiobiose, D-turanose, D-lyxose, D-tagatose, D-fucose, L-fucose, D-arabitol, L-arabitol, gluconate, 2-ketogluconate, 5-ketogluconate.

12. Method according to one of claims 6 to 11, wherein a texturizing agent is chosen from those of the following group: agar, agarose, gelatine, xanthan, scleroglucan, guar gum.

13. Method according to any one of the preceding claims,
wherein the indicator is inactivated when it is not associated with the product and activated when it is associated with the product.

14. Method according to claim 13, wherein the inactivation can be realized with the aid of means chosen from the following group: freezing, microencapsulation of the microbial substance and/or the nutrients, compartmentalization.

15. Method according to one of claims 13 or 14, wherein the activation of the indicator is realized by a physical action chosen from the following group:
variation in pressure, variation in temperature,
variation in the wavelength of exposure to radiation.

16. Method according to any one of claims 1 to 15, wherein the microbial substance is frozen or thawed.

17. Application of the method according to any one of the preceding claims to products of a food or biological or pharmaceutical type.

18. Application of the method according to any one of claims 1 16 to food products wherein the parameter of the indicator is such that the appearance of the signal indicating a change of state is later the closer the preservation temperature of the product is to the optimum preservation temperature.

19. Application of the method according to any one of claims 1 to 16 to a food product which is to be preserved at temperatures of between about 0 °C and +4 °C, and wherein the indicator comprises a microbial substance having an acidifying activity at temperatures of less than or equal to about +4 °C.

20. Device for determining whether or not a product is in a condition for use or consumption, this product being intended for preservation below a limit temperature under temperature conditions and for a period of time which limits its degradation, this device comprising at least one indicator which is to be associated with the product, this indicator being such that it provides a signal indicating the possibility of use or consumption of the product and then, after expiry of a preservation period of time, a signal indicating a change of state of the possibility of use or consumption of the product, this period of time evolving as a function of the conditions of preservation of the product,
the indicator being such that it provides information which enables the product to be used or consumed for a longer period of time if the product has been preserved under conditions better than the standard or recommended conditions, or for a shorter period of time if the product has been preserved under conditions poorer than the standard or recommended conditions,
the indicator containing at least one microbial substance having an acidifying activity which varies the pH of the medium containing it,
the said microbial substance containing at least one strain of bacteria chosen from:
- the strain CRYO-CB001 of the species *Carnobacterium piscicola* registered at the CNCM (Collection Nationale de Culture de Microorganismes, Paris, France) under no. CNCM 1-3297 on 16th September 2004; and
- the strain CRYO-CB002 of the species *Carnobacterium piscicola* registered at the CNCM (Collection Nationale de Culture de Microorganismes, Paris, France) under no. CNCM 1-3298 on 16th September 2004;
the said indicator also containing a developer and means for providing a signal indicating the change in condition when the pH of the medium containing the microbial substance falls below a predetermined value.

21. Device according to claim 20, wherein the developer is such that it forms a precipitate when the pH falls below the predetermined value.

22. Device according to claim 21, wherein the developer comprises casein.

23. Device according to one of claims 20 or 21, wherein the developer comprises at least one coloured indicator.

24. Device according to claim 23, wherein the coloured indicator is included in the following group:
alizarin, alizarin sodium sulfonate, alizarin red, benzopurpurin, benzoylauramin G, benzoylethylauramin, resorcinol blue, bromochlorophenol blue, bromocresol green, bromophenol blue, carmine acid, 2,4-dinitrophenol, 4-(4-dimethylamino-1-naphthylazo)-3-methoxybenzenesulfonic acid, α-dinitrophenol, β-dinitrophenol, γ-dinitrophenol, disodium 4,4-bis(o-tolyltriazeno)-2,2'-stilbenedisulfonate, disodium 4,4-bis(p-dimethylaminophenylazo)-2,2'-stilbenedisulfonate, 4-(p-ethoxyphenylazo)-m-phenylenediamine monohydrochloride, ethyl red, ethyl orange, hexamethoxy red, hydroquinol sulfonaphthalein, lacmoid, iodeosin, lasmoid, N,N-dimethyl-p-(m-tolylazo)aniline, 4'-methoxy-2,4-diaminoazobenzene, methyl red, methyl red alphazurin, α-naphthylamine, α-naphthylaminoazobenzene, naphthyl red, oxime blue, 4'-oxy-3'-methyl-2,4-diaminoazobenzene, 4'-oxy-2,4-diaminoazobenzene, 4-phenylazo-1-naphthylamine, parafuchsin-hexaacetic acid, paranitrophenol, p-ethoxychrysoidine, p-sulfo-o-methoxybenzeneazodimethyl-α-naphthylamine, Congo red, red violet, resazurin, naphthyl red, tetrabromocresol, tetraiodophenolsulfophthalein, aurin acid, dithiozone, chromium blue, bromocresol purple, indigo carmine, cacotheline, calcein, eosin extra, eriochromocyanine,
thiazole yellow, eriochrome black T, α-nitro-β-naphthol, orange III, methyl red, rhodamine B,
potassium rhodizonate, thymolsulfonephthalein, rhodium chloride, 2,6,7-trihydroxy-9-phenylisoxanthene, thorin, xylene orange tetrasodium salt, anthocyanes, phenolphthalein, benzopurpurin 4B, benzopurpurin B,
alpha-naphthyl red hydrochloride, litmus, methyl red, mixed indicator, lacmoid.

25. Device according to any one of claims 20 to 24,
wherein at least one of the parameters of the indicator is such that the indicator is adapted to the product, the parameters being included in the following group: the nature of the microbial substance, the amount of this microbial substance, the nature of the nutrients, the amount of these nutrients, the nature of the elements necessary for production of acid by the microbial substance and the amount of these elements, the nature of a texturizing agent of the medium containing the microbial substance and the amount of this texturizing agent, the starting pH of the medium, parameters which influence the water activity of the medium, the nature of the developer used to measure the drop in pH, and the amount of this developer, parameters which determine the pH at which this developer changes condition.

26. Device according to claim 25, wherein the indicator is such that the parameters are such that the signal indicating the change of state takes place after a predetermined period of time when the product is preserved under standard or recommended conditions.

27. Device according to claim 25, comprising an indicator
wherein the parameters are such that the signal indicating the change of state appears when the product can no longer be consumed or used.

28. Device according to any one of claims 25 to 27,
wherein the nutrients include a source of carbon and a source of nitrogen.

29. Device according to any one of claims 20 to 28,
**characterized in that** it contains a culture medium containing a source of carbon and a source of nitrogen.

30. Device according to one of claims 28 or 29, wherein the source of carbon is chosen from the sugars of following group: glycerol, erythritol, D-arabinose, L-arabinose, ribose, D-xylose, L-xylose, adonitol, methyl xyloside, galactose, D-glucose, D-fructose, D-mannose, L-sorbose, rhamnose, dulcitol, inositol, mannitol, sorbitol, α-methyl D-mannoside, α-methyl D-glucoside, N-acetylglucosamine, amygdalin, arbutin, esculin, salicin, cellobiose, maltose, lactose, melibiose, saccharose, trehalose, inulin, melezitose, D-raffinose, starch, glycogen, xylitol, β-gentiobiose, D-turanose, D-lyxose, D-tagatose, D-fucose, L-fucose, D-arabitol, L-arabitol, gluconate, 2-ketogluconate, 5-ketogluconate.

31. Device according to any one of claims 25 to 30,
wherein a texturizing agent is included among those of the following group: agar, agarose, gelatine, xanthan, scleroglucan, guar gum.

32. Device according to any one of claims 1 to 31,
**characterized in that** it comprises a texturizing agent chosen from the following group: agar, agarose, gelatine, xanthan, scleroglucan, guar gum.

33. Device according to any one of claims 20 to 32,
wherein the indicator is such that it is inactivated when it is not associated with the product and activated when it is associated with the product.

34. Device according to claim 33, such that the inactivation can be realized with the aid of means included in the following group: freezing, microencapsulation of the microbial substance and/or the nutrients, compartmentalization.

35. Device according to one of claims 33 or 34, such that the activation of the indicator is realized by a physical action chosen from the following group:
variation in pressure, variation in temperature,
variation in the wavelength of exposure to radiation.

36. Device according to one of claims 20 to 35, wherein the microbial substance can be frozen and thawed.

37. Device according to one of claims 20 to 36, wherein the indicator is present in the form of a label, preferably a self-adhesive label.

38. Device according to claim 37, wherein the indicator comprises at least one surface with a zone, which allows observation of the signal produced by the developer.

39. Device according to one of claims 37 or 38, wherein the label comprises several internal compartments, for example capsules, which allow separation of one or more constituents of the component from others for a given period of time, it being possible for the walls of the said compartments to be broken by any suitable means, for example by pressure on the label.

## Patentansprüche

1. Verfahren zur Bestimmung, ob ein Produkt zur Verwendung oder zum Verbrauch geeignet ist, wobei dieses Produkt unterhalb einer Grenztemperatur aufbewahrt werden soll, unter Temperaturbedingungen und während einer Zeitdauer, die sein Verderben begrenzen, wobei unter diesem Verfahren dem Produkt ein Indikator von der Art zugeordnet wird, die ein Signal abgibt, das die Möglichkeit der Verwendung oder des Verbrauchs des Produktes anzeigt und, nach Ablauf einer Aufbewahrungszeit, ein Signal, das einen Wechsel des Zustandes der Verwendungs- oder der Verbrauchsmöglichkeit des Produktes anzeigt, wobei sich diese Zeitdauer als Funktion der Konservierungsbedingungen des Produktes ändert,
wobei der Indikator von der Art ist, die eine Information bereitstellt, nach der die Möglichkeit gegeben ist, das Produkt während eines längeren Zeitraumes zu verwenden oder zu verbrauchen, wenn das Produkt unter günstigeren Bedingungen aufbewahrt wurde, als die Standardbedingungen oder die vorgeschriebenen Bedingungen, oder während eines kürzeren Zeitraums, wenn das Produkt unter ungünstigeren Bedingungen als die Standardbedingungen oder die vorgeschriebenen Bedingungen aufbewahrt wurde,
wobei der Indikator mindestens einen mikrobiellen Stoff enthält, der eine säurebildende Aktivität aufweist, die eine Variation des pH-Wertes des Milieus, in dem sie sich befindet, bewirkt,
wobei der besagte mikrobielle Stoff mindestens einen Bakterienstamm enthält, der gewählt wurde aus:
- dem Stamm CRYO-CB001 der Spezies *Carnobacterium piscicola,* der beim CNCM (Nationale Sammlung für die Kultur von Mikroorganismen, Paris, Frankreich) unter der Nummer CNCM 1-3297 am 16. September 2004 registriert wurde, und
- dem Stamm CRYO-CB002 der Spezies *Carnobacterium piscicola,* der beim CNCM (Nationale Sammlung für die Kultur von Mikroorganismen, Paris, Frankreich) unter der Nummer CNCM I-3298 am 16. September 2004 registriert wurde;
in dem mit Hilfe einer enthüllenden Substanz das Signal erzeugt wird, das den Zustandswechsel anzeigt, wenn der pH-Wert des Milieus, in dem sich der mikrobielle Stoff befindet, unter einen vorgegebenen Wert fällt.

2. Verfahren nach Anspruch 1, wobei die enthüllende Substanz ein Präzipitat bildet, wenn der pH-Wert unterhalb des vorgegebenen Wertes fällt.

3. Verfahren nach Anspruch 2, wobei die enthüllende Substanz Kasein enthält.

4. Verfahren nach einem der Ansprüche 1 oder 2, wobei die enthüllende Substanz mindestens einen Farbstoffindikator enthält.

5. Verfahren nach Anspruch 4, wobei der Farbstoffindikator aus folgender Gruppe ausgewählt wird: Alizarin, Alizarin-Natrium-Sulfonat, Alizarinrot, Benzo-Purpurin, BenzoylAuramin G, Benzoyl-Ethyl-Auramin, Resorzinblau, Bromchlorphenolblau, Bromkresolgrün, Bromphenolblau, Karminsäure, 2,4 Dinitrophenol, 4- (4-Dimethylamino-l-Naphtyl-Stickstoff)-3-Methoxybenzen-Sulfonsäure, α- Dinitrophenol, β- Dinitrophenol, γ- Dinitrophenol, Dinatrium4,4- Bis (O-Tolytriazeno)-2, 2'- Stilbendisulfonat, Dinatrium4,4- Bis (P-Dimethylaminophenylazo)-2 , 2'- Stilbendisulfonat, 4-(P-Ethoxyphenylazo)-M-PhenylenDiamin Monohydrochlorid, Ethylrot, Ethylorange, Hexamethoxyrot, Hydrochinol-Sulfonaphtalein, Lacmoid, lodeosin, Lasmoid, N,Ndimethyl-P- (M-Tolylazo) Anilin, 4'-Methoxy-2. 4-Diaminoazobenzen, Methylrot, Methylrot- Alphazurin, α-Naphtylamin, α-Naphtylaminoazo-Benzen, Naphtylrot, Oximblau, 4'-Oxy-3'-Methyl-2. 4-Diaminoazobenzen, 4'-Oxy- 2.4-Diaminoazobenzen, 4-Phenylazo-1-Naphtylamin, Parafuchsin- Hexa-Essigsäure, Paranitrophenol, P-Ethoxychryso-lod, P-Sulfo- O-Methoxybenzenazodimethyl-α-Naphtylamin, Kongorot, Kardinalrot, Resazurin, Naphtylrot, Tetrabrom-Kresol, Tetraiod-Phenolsulfophtalein, Aurinsäure, Dithiozon, Chromblau, Bromkresolpurpur, Indigo karmin, Cakothelin, Calzein, Eosin extra, Eriochromzyanin, Thiazolgelb, Eriochrom-T-Schwarz, α-Nitro β-Naphtol, Orange III, Methylrot, Rhodamin B, Kaliumrhodizonat Thymolsulffonephtalein, Rhodiumcholrid, Trihydroxy2, 6, 7 Phenyl-9-lsoxanthen, Thorin, Xylen Orange Tetranatriumsalz, Anthozyane, Phenolphtalein, Benzopurpurin 4B, Benzopurpurin B, Alphanaphtylrot-Hydrochlorid, Lackmus, Methylrot, Gemischter Indikator, Lackmoid.

6. Verfahren nach einem der Ansprüche 1 bis 5, wobei, um den Indikator an das Produkt anzupassen, man mindestens einen der Parameter aus der folgenden Gruppe wählt:
die Art des mikrobiellen Stoffs, die Menge dieses mikrobiellen Stoffs, die Art der Nährstoffe,
die Menge dieser Nährstoffe, die Art der zum Erzeugen von Säure durch den mikrobiellen Stoff erforderlichen Elemente sowie die Menge dieser Elemente, die Art eines Strukturfestigers des Milieus, in dem sich der mikrobielle Stoff befindet und die Menge dieses Strukturfestigers, der anfängliche pH-Wert des Milieus, die Parameter, welche die Wasseraktivität des Milieus beeinflussen, die Art der verwendeten enthüllenden Substanz,
um den Abfall des pH-Wertes zu messen und die Menge dieser enthüllenden Substanz sowie die den pH-Wert, bei dem diese enthüllende Substanz ihren Zustand wechselt, bestimmenden Parameter.

7. Verfahren nach Anspruch 6, wobei die Parameter derart gewählt werden, dass das Signal zum Anzeigen des Zustandswechsels am Ende eines vorgegebenen Zeitraums auftritt, wenn das Produkt unter Standardbedingungen oder unter vorgeschriebenen Bedingungen aufbewahrt wird.

8. Verfahren nach Anspruch 6, wobei die Parameter derart gewählt werden, dass das Signal zum Anzeigen des Zustandswechsels dann auftritt, wenn das Produkt nicht mehr zum Verbrauch geeignet ist.

9. Verfahren nach einem der Ansprüche 7 oder 8, wobei unter Standardbedingungen oder vorgeschriebenen Bedingungen, die Parameter des Indikators derart gewählt werden, dass das Signal zum Anzeigen des Zustandswechsels nach einem vorgegebenen Zeitraum auftritt, wobei dieser Zeitraum durch Änderung einer dieser Parameter verlängert wird, indem dieser Parameter von den optimalen Säuerungswerten durch den mikrobiellen Stoff entfernt wird.

10. Verfahren nach einem der Ansprüche 6 bis 9, wobei die Nährstoffe eine Kohlenstoffquelle und eine Stickstoffquelle sowie, bevorzugterweise, Mineralsalze und/oder Vitamine und/oder Spurenelemente enthalten.

11. Verfahren nach Anspruch 10, wobei die Kohlenstoffquelle unter den Zuckern der folgenden Gruppe gewählt wird: Glyzerin, Erythritol, D-Arabinose, L-Arabinose, Ribose, D-Xylose, L-Xylose, Ribitol, Methyl-Xylosid, Galaktose, D-Glukose, D-Fruktose, D-Mannose, L-Sorbose, Rhamnose, Dulzitol, Inositol, Mannit, Sorbit, αMethyl-D-Mannosid, (α Methyl-D-Glukosid), N Azetyl Glukosamin, Amygdalin, Arbutin, Aesculin, Salizin, Zellobiose, Maltose, Laktose, Melibiose, Saccharose, Trehalose, Inulin, Raffinose, D-Raffinose, Stärke, Glykogen, Xylit, β Gentiobiose, D-Turanose, D-Lyxose, D-Tagatose, D-Fucose, L-Fucose, D-Arabitol, L-Arabitol, Gluconat, 2 Ketogluconat, 5 Ketogluconat.

12. Verfahren nach einem der Ansprüche 6 bis 11, wobei ein Strukturfestiger aus der folgenden Gruppe gewählt wird: japanische Gelatine, Agarose, Gelatine, Xanthan, Skleroglukan, Guar Gum.

13. Verfahren nach einem der vorhergehenden Ansprüche, wobei der Indikator inaktiviert ist, wenn er nicht mit dem Produkt assoziiert ist und aktiviert wird, wenn er mit dem Produkt assoziiert ist.

14. Verfahren nach Anspruch 13, wobei die Inaktivierung mit Hilfe von Mitteln erfolgen kann, die aus der folgenden Gruppe gewählt werden: Einfrieren, Mikroeinkapselung des mikrobiellen Stoffs und/oder der Nährstoffe, Auftrennen nach Fächern.

15. Verfahren nach einem der Ansprüche 13 oder 14, wobei die Aktivierung des Indikators durch eine aus der folgenden Gruppe gewählten physischen Aktion erfolgt:
Druckvariation, Temperaturvariation, Variation der Wellenlänge bei Bestrahlung.

16. Verfahren nach einem der Ansprüche 1 bis 15, wobei der mikrobielle Stoff eingefroren oder aufgetaut wird.

17. Anwendung des Verfahrens nach einem der vorhergehenden Ansprüche auf Produkte aus dem Bereich der Lebensmittel, der biologischen Produkte oder der pharmazeutischen Produkte.

18. Anwendung des Verfahrens nach einem der Ansprüche 1 bis 16 auf Lebensmittel, wobei der Indikator derart parametrisiert wird, dass das Erscheinen des eine Zustandsänderung anzeigenden Signals umso mehr verzögert wird, je näher die Aufbewahrungstemperatur des Produktes bei der optimalen Konservierungstemperatur liegt.

19. Anwendung des Verfahrens nach einem der Ansprüche 1 bis 16 auf ein Lebensmittel, welches bei Temperaturen zwischen 0 °C und etwa +4 °C aufbewahrt werden soll, wobei der Indikator einen mikrobiellen Stoff enthält, der bei Temperaturen unterhalb oder bei ca. +4 °C eine säurebildende Aktivität aufweist.

20. Vorrichtung zum Feststellen, ob ein Produkt zur Verwendung oder zum Verbrauch geeignet ist, wobei dieses Produkt bei Temperaturen unterhalb einer Grenztemperatur, unter Temperaturbedingungen und während eines Zeitraums aufbewahrt werden soll, die sein Verderben einschränken, wobei diese Vorrichtung mindestens einen Indikator enthält, der in der Lage ist, die Möglichkeit der Verwendung oder des Verbrauchs des Produktes anzuzeigen und wobei nach Ablauf eines Aufbewahrungszeitraums, ein Signal erscheint, das eine Zustandsänderung bezüglich der Möglichkeit der Verwendung oder des Verbrauchs des Produktes anzeigt, wobei dieser Zeitraum als Funktion der Konservierungsbedingungen des Produktes variiert,
wobei der Indikator derart ist, dass er eine Information liefert, die eine Verwendung oder einen Verbrauch des Produktes während eines längeren Zeitraums ermöglicht, wenn das Produkt unter günstigeren Bedingungen als die Standardbedingungen oder die vorgeschriebenen Bedingungen, bzw. während eines kürzeren Zeitraums, wenn das Produkt unter ungünstigeren Bedingungen als die Standardbedingungen oder die vorgeschriebenen Bedingungen aufbewahrt wurde,
wobei der Indikator mindestens einen mikrobiellen Stoff enthält, der eine säurebildende Aktivität aufweist, die eine Variierung des pH-Wertes des Milieus indem er sich befindet bewirkt,
wobei der besagte mikrobielle Stoff mindestens einen Bakterienstamm enthält, der ausgewählt wurde aus:
- dem Stamm CRYO-CB001 der Spezies Camobacterium piscicola, der beim CNCM (Nationale Sammlung für die Kultur von Mikroorganismen, Paris, Frankreich) unter der Nummer CNCM 1-3297 am 16. September 2004 registriert wurde und,
- dem Stamm CRYO-CB002 der Spezies *Camobacterium piscicola,* der beim CNCM (Nationale Sammlung für die Kultur von Mikroorganismen, Paris, Frankreich) unter der Nummer CNCM 1-3298 am 16. September 2004 registriert wurde,
wobei der besagte Indikator darüber hinaus eine enthüllende Substanz und Mittel enthält, um ein Signal zum Anzeigen der Zustandsänderung bereitzustellen, wenn der pH-Wert des Milieus, indem sich der mikrobielle Stoff befindet unterhalb eines vorgegebenen Wertes fällt.

21. Vorrichtung nach Anspruch 20, wobei die enthüllende Substanz derart ist, dass sie ein Präzipitat bildet, wenn der pH-Wert unterhalb des vorgegebenen Wertes fällt.

22. Vorrichtung nach Anspruch 21, wobei die enthüllende Substanz Kasein enthält.

23. Vorrichtung nach einem der Ansprüche 20 oder 21, wobei die enthüllende Substanz mindestens einen Farbstoffindikator enthält.

24. Vorrichtung nach Anspruch 23, wobei der Farbstoffindikator aus folgender Gruppe ausgewählt wird: Alizarin, Alizarin-Natrium-Sulfonat, Alizarinrot, Benzo-Purpurin, BenzoylAuramin G, Benzoyl-Ethyl-Auramin, Resorzinblau, Bromchlorphenolblau, Bromkresolgrün, Bromphenolblau, Karminsäure, 2,4 Dinitrophenol, 4- (4-Dimethylamino-1-Naphtyl-Stickstoff)-3-Methoxybenzen-Sulfonsäure, α- Dinitrophenol, β- Dinitrophenol, γ- Dinitrophenol, Dinatrium4,4- Bis (O-Tolytriazeno)-2, 2'- Stilbendisulfonat, Dinatrium4,4- Bis (P-Dimethylaminophenylazo)-2 , 2'- Stilbendisulfonat, 4-(P-Ethoxyphenylazo)-M-PhenylenDiamin Monohydrochlorid, Ethylrot, Ethylorange, Hexamethoxyrot, Hydrochinol-Sulfonaphtalein, Lacmoid, Iodeosin, Lasmoid, N,Ndimethyl-P- (M-Tolylazo) Anilin, 4'-Methoxy-2. 4-Diaminoazobenzen, Methylrot, Methylrot- Alphazurin, α-Naphtylamin, α-Naphtylaminoazo-Benzen, Naphtylrot, Oximblau, 4'-Oxy-3'-Methyl-2. 4-Diaminoazobenzen, 4'-Oxy- 2.4-Diaminoazobenzen, 4-Phenylazo-1-Naphtylamin, Parafuchsin- Hexa-Essigsäure, Paranitrophenol, P-Ethoxychryso-lod, P-Sulfo- O-Methoxybenzenazodimethyl-a-Naphtylamin, Kongorot, Kardinalrot, Resazurin, Naphtylrot, Tetrabrom-Kresol, Tetraiod-Phenolsulfophtalein, Aurinsäure, Dithiozon, Chromblau, Bromkresolpurpur, Indigo karmin, Cakothelin, Calzein, Eosin extra, Eriochromzyanin, Thiazolgelb, Eriochrom-T-Schwarz, α-Nitro β-Naphtol, Orange III, Methylrot, Rhodamin B, Kaliumrhodizonat, Thymolsulffonephtalein, Rhodiumcholrid, Trihydroxy2, 6, 7 Phenyl-9-Isoxanthen, Thorin, Xylen Orange Tetranatriumsalz, Anthozyane, Phenolphtalein, Benzopurpurin 4B, Benzopurpurin B, Alphanaphtylrot-Hydrochlorid, Lackmus, Methylrot, Gemischter Indikator, Lackmoid.

25. Vorrichtung nach einem der Ansprüche 20 bis 24, wobei mindestens einer der Parameter des Indikators derart ist, dass der Indikator dem Produkt angepasst ist, wobei die Parameter aus der folgenden Gruppe stammen: die Art des mikrobiellen Stoffs, die Menge dieses mikrobiellen Stoffs, die Art der Nährstoffe, die Menge dieser Nährstoffe, die Art der zum Erzeugen von Säure durch den mikrobiellen Stoff erforderlichen Elemente sowie die Menge dieser Elemente, die Art eines Strukturfestigers des Milieus, in dem sich der mikrobielle Stoff befindet und die Menge dieses Strukturfestigers, der anfängliche pH-Wert des Milieus, die Parameter, welche die Wasseraktivität des Milieus beeinflussen, die Art der verwendeten enthüllenden Substanz, um den Abfall des pH-Wertes zu messen und die Menge dieser enthüllenden Substanz sowie die den pH-Wert, bei dem diese enthüllende Substanz ihren Zustand wechselt, bestimmenden Parameter.

26. Vorrichtung nach Anspruch 25, wobei der Indikator derart ist, dass die Parameter derart sind, dass das Signal zum Anzeigen des Zustandswechsels am Ende eines vorgegebenen Zeitraums auftritt, wenn das Produkt unter Standardbedingungen oder unter vorgeschriebenen Bedingungen aufbewahrt wird.

27. Vorrichtung nach Anspruch 25, die einen Indikator aufweist, in dem die Parameter derart sind, dass das Signal zum Anzeigen des Zustandswechsels dann auftritt, wenn das Produkt nicht mehr zur Verwendung oder zum Verbrauch geeignet ist.

28. Vorrichtung nach einem der Ansprüche 25 bis 27, wobei die Nährstoffe eine Kohlenstoffquelle und eine Stickstoffquelle enthalten.

29. Vorrichtung nach einem der Ansprüche 20 bis 28, **dadurch gekennzeichnet, dass** sie ein Kulturmedium umfasst, das eine Kohlenstoffquelle und eine Stickstoffquelle enthält.

30. Vorrichtung nach einem der Ansprüche 28 oder 29, wobei die Kohlenstoffquelle unter den Zuckern der folgenden Gruppe gewählt wird: Glyzerin, Erythrit, D-Arabinose, L-Arabinose, Ribose, D-Xylose, L-Xylose, Ribitol, Methyl-Xylosid, Galaktose, D-Glukose, D-Fruktose, D-Mannose, L-Sorbose, Rhamnose, Dulzitol, Inositol, Mannit, Sorbit, αMethyl-D-Mannosid, (α Methyl-D-Glukosid), N Azetyl Glukosamin, Amygdalin, Arbutin, Aesculin, Salizin, Zellobiose, Maltose, Laktose, Melibiose, Saccharose, Trehalose, Inulin, Raffinose, D-Raffinose, Stärke, Glykogen, Xylit, β Gentiobiose, D-Turanose, D-Lyxose, D-Tagatose, D-Fucose, L-Fucose, D-Arabitol, L-Arabitol, Gluconat, 2 Ketogluconat, 5 Ketogluconat.

31. Vorrichtung nach einem der Ansprüche 25 bis 30, wobei ein Strukturfestiger aus der folgenden Gruppe enthalten ist: japanische Gelatine, Agarose, Gelatine, Xanthan, Skleroglukan, Guar Gum.

32. Vorrichtung nach einem der Ansprüche 1 bis 31, **dadurch gekennzeichnet, dass** sie einen Strukturfestiger enthält, der aus der folgenden Gruppe gewählt wird: japanische Gelatine, Agarose, Gelatine,Xanthan, Skleroglukan, Guar Gum.

33. Vorrichtung nach einem der Ansprüche 20 bis 32, wobei der Indikator derart ist, dass er inaktiviert ist, wenn er nicht mit dem Produkt assoziiert ist und aktiviert wird, wenn er mit dem Produkt assoziiert ist.

34. Vorrichtung nach Anspruch 33, wobei die Inaktivierung mit Hilfe von Mitteln erfolgen kann, die aus der folgenden Gruppe gewählt werden: Einfrieren, Mikroeinkapselung des mikrobiellen Stoffs und/oder der Nährstoffe, Auftrennen nach Fächern.

35. Vorrichtung nach einem der Ansprüche 33 oder 34, wobei die Aktivierung des Indikators durch eine aus der folgenden Gruppe gewählten physischen Aktion erfolgt:
Druckvariation, Temperaturvariation, Variation der Wellenlänge bei Bestrahlung.

36. Vorrichtung nach einem der Ansprüche 20 bis 35, wobei der mikrobielle Stoff eingefroren oder aufgetaut werden kann.

37. Vorrichtung nach einem der Ansprüche 20 bis 36, wobei der Indikator die Form eines Etiketts und bevorzugterweise eines selbstklebenden Etiketts aufweist.

38. Vorrichtung nach Anspruch 37, wobei der Indikator mindestens eine Fläche aufweist, mit einem Bereich, der das Beobachten des von der enthüllenden Substanz erzeugten Signals ermöglicht.

39. Vorrichtung nach einem der Ansprüche 37 oder 38, wobei das Etikett mehrere innere Fächer, beispielsweise Kapseln, aufweist, die es ermöglichen, ein Bestandteil oder mehrere Bestandteile der Zusammensetzung von den anderen während eines vorgegebenen Zeitraums zu trennen, wobei sich die Wände der besagten Fächer durch ein geeignetes Mittel, beispielsweise durch Ausüben eines Drucks auf das Etikett, brechen lassen.
